# EUROPÄISCHE PATENTSCHRIFT

(11) **EP 2 041 719 B1**
(45) Veröffentlichungstag und Bekanntmachung des Hinweises auf die Patenterteilung: **20.10.2010**
(21) Anmeldenummer: 07726100.6
(22) Anmeldetag: 21.06.2007
(51) Int. Cl.: G06T 7/00

(54) **VERFAHREN ZUM BESTIMMEN EINER EIGENSCHAFTSKARTE FÜR EINEN GEGENSTAND, INSBESONDERE FÜR EIN LEBEWESEN, BASIEREND AUF ZUMINDEST EINEM ERSTEN BILD, INSBESONDERE EINEM KERNSPINRESONANZBILD**
METHOD FOR DETERMINING A PROPERTY MAP FOR AN OBJECT IN PARTICULAR FOR A LIVING BEING BASED ON AT LEAST ONE FIRST IMAGE IN PARTICULAR A NUCLEAR MAGNETIC RESONANCE IMAGE
PROCÉDÉ DE DÉTERMINATION D'UNE CARTE DE PROPRIÉTÉ POUR UN OBJET, EN PARTICULIER POUR UN ÊTRE HUMAIN, BASÉ SUR AU MOINS UNE PREMIÈRE IMAGE, EN PARTICULIER UNE IMAGE PAR RÉSONANCE MAGNÉTIQUE NUCLÉAIRE

(30) Priorität: 12.07.2006 DE 102006033383
(43) Veröffentlichungstag der Anmeldung: 01.04.2009
(73) Patentinhaber: Eberhard-Karls-Universität Tübingen, 72076 Tübingen (DE); Max-Planck-Gesellschaft zur Förderung der Wissenschaften e.V., 80539 München (DE)
(72) Erfinder: PICHLER, Bernd, 85298 Scheyern (DE); HOFMANN, Matthias, 72076 Tübingen (DE); SCHÖLKOPF, Bernhard, 72076 Tübingen (DE); STEINKE, Florian, 71083 Herrenberg (DE)
(74) Vertreter: Wegener, Markus
(86) Internationale Anmeldenummer: PCT/EP2007/005455
(87) Internationale Veröffentlichungsnummer: WO 2008/006451

(56) Entgegenhaltungen:
- US-A1- 2002 165 837
- ZHANG X ET AL: "Application of support vector machines in classification of magnetic resonance images" INTERNATIONAL JOURNAL OF COMPUTERS & APPLICATIONS ACTA PRESS USA, Bd. 28, Nr. 2, April 2006 (2006-04), Seiten 122-128, XP002450169 ISSN: 1206-212X
- CHAN IAN ET AL: "Detection of prostate cancer by integration of line-scan diffusion, T2-mapping and T2-weighted magnetic resonance imaging" MEDICAL PHYSICS, AIP, MELVILLE, NY, US, Bd. 30, Nr. 9, September 2003 (2003-09), Seiten 2390-2398, XP012012225 ISSN: 0094-2405
- TAN OU ET AL: "Automatic segmentation and classification of human brain images based on TT atlas" ENGINEERING IN MEDICINE AND BIOLOGY SOCIETY, 1998. PROCEEDINGS OF THE 20TH ANNUAL INTERNATIONAL CONFERENCE OF THE IEEE HONG KONG, CHINA 29 OCT.-1 NOV. 1998, PISCATAWAY, NJ, USA,IEEE, US, Bd. 2, 29. Oktober 1998 (1998-10-29), Seiten 700-702, XP010320610 ISBN: 0-7803-5164-9
- ZAIDI HABIB ET AL: "Magnetic resonance imaging-guided attenuation and scatter corrections in three-dimensional brain positron emission tomography" MEDICAL PHYSICS, AIP, MELVILLE, NY, US, Bd. 30, Nr. 5, Mai 2003 (2003-05), Seiten 937-948, XP012012081 ISSN: 0094-2405

## Beschreibung

Die Erfindung betrifft ein Verfahren zum Bestimmen einer Eigenschaftskarte für einen Gegenstand, insbesondere für ein Lebewesen, basierend auf zumindest einem ersten Bild, insbesondere einem Kernspinresonanzbild, MR-Bild.

Das erfindungsgemäße Verfahren soll u.a. bei einer Auswertung von Positronenemissionstomographiebildern, PET-Bildern zum Einsatz kommen, die mittels eines zurzeit in der Entwicklung befindlichen PET-MR-Scanners aufgenommen werden. Dieser PET-MR-Scanner erlaubt eine Aufnahme eines PET- und eines MR-Bildes, wobei beide Bilder gleichzeitig oder zeitlich beabstandet aufgenommen werden und einen etwa gleichen Bildausschnitt darstellen. Das (unkorrigierte) PET-Bild soll mit Hilfe einer Schwächungskarte korrigiert werden, damit das korrigierte, quantitativ präzise PET-Bild anschließend bspw. in der Medizindiagnostik verwendet werden kann. Eine Berechung einer Schwächungskarte basierend auf MR-Bildern ist eine mögliche Anwendung des Verfahrens der vorliegenden Erfindung.

PET ist ein bildgebendes Verfahren, das in der Nuklearmedizin zur Erzeugung eines Schnittbildes von lebenden Organismen, beispielsweise Menschen oder Tieren, verwendet wird. Das aufgenommene PET-Bild stellt eine "funktionelle" Abbildung eines Körperabschnitts des lebenden Organismus dar, da das Bild über biochemische und physiologische Vorgänge, wie bspw. Stoffwechselprozesse eines Organs, Aufschluss gibt.

Um ein PET-Bild zu erzeugen, wird dem zu untersuchenden Organismus ein Radiopharmakum, beispielsweise ¹⁵O oder ¹⁸F, mit einer Halbwertszeit im Bereich von z.B. 20 Minuten verabreicht. Bei einem Zerfall von Radiopharmaka werden Positronen emittiert (β⁺-Zerfall), die im Körper nach kurzer Distanz mit Elektronen annihilieren und pro Elektron-Positron-Annihilation zwei Gammaquanten, γ-Quanten, erzeugen. Diese beiden γ-Quanten entfernen sich etwa unter 180° voneinander und treffen bei gegenüberliegenden Positionen eines ringförmigen Detektors auf. Das Schnittbild des zu untersuchenden Körperabschnitts entsteht durch eine Vielzahl solcher Koinzidenzregistrierungen in dem Detektor.

Für ein quantitativ genaues PET-Bild müssen zwei durch die Aufnahmetechnik bedingte Effekte berücksichtigt werden. Die durch die Elektron-Positron-Annihilation auftretenden γ-Quanten erfahren infolge einer Absorption eine Abschwächung im Körper. Ferner wird die Detektion der γ-Quanten durch im Körper auftretende Vielfachstreuprozesse der γ-Quanten, beispielsweise Comptonstreuung, beeinflusst. Der Anteil der gestreuten γ-Quanten hängt von der γ-Energie des verwendeten radioaktiven Isotops, der Dichte der zu durchquerenden Schicht und deren Schwächungswert ab.

Eine Schwächungs- und Streukorrektur des aufgenommenen PET-Bildes korrigiert die beiden Effekte zumindest teilweise. Eine bekannte Korrektur für ein gesamtes PET-Bild wird mit Hilfe einer so genannten Schwächungskarte durchgeführt, die punktweise Korrekturwerte für das PET-Bild bereitstellt, um Abschwächungs- und Streueffekte der γ-Quanten im Körper rechnerisch zu berücksichtigen.

MR ist ein weiteres bildgebendes Verfahren zur Erzeugung eines Schnittbildes eines zu untersuchenden Körperabschnitts. Bei diesem Verfahren werden die (transversalen und longitudinalen) Relaxationszeiten T₁ und T₂ von durch Hochfrequenz-Impulse angeregten Kernspins gemessen. Das MR-Bild enthält Informationen über anatomische Strukturen des zu untersuchenden Körperabschnitts, da MR einen besonders hohen Kontrast zwischen Gewebe, Knochen und Weichteilen bietet. Dieser Kontrast ist durch die verschiedene Signalintensität der einzelnen Bildpunkte bestimmt, die im MR-Bild einem Grauwert bzw. einer Helligkeitsstufe entspricht.

Ein bekanntes, zurzeit üblicherweise verwendetes Verfahren, eine Schwächungs- und Streukorrektur für ein PET-Bild durchzuführen, verwendet ein zusätzliches Computertomographie-Bild, CT-Bild, das mittels eines PET-CT-Scanners aufgenommen wird.

Ein CT-Bild wird mittels CT (Computertomographie), einem weiteren bildgebenden Verfahren, erzeugt, und es basiert auf mehreren Röntgenaufnahmen eines zu untersuchenden Körperabschnitts. Diese Transmissionsaufnahmen werden rechnergestützt zu einem dreidimensionalen Modell verarbeitet, so dass beliebige Schnittbilder des untersuchten Körperabschnitts rekonstruiert werden können. Verschiedene Graustufen des CT-Bildes entsprechen verschieden stark abgeschwächten Signalintensitäten und geben Aufschluss über eine Dichteverteilung des Körperabschnitts, insbesondere die Anordnung von Knochen in dem Körperabschnitt.

Um eine Schwächungs- und Streukorrektur basierend auf einem CT-Bild durchzuführen, werden aus dem CT-Bild punktweise Schwächungswerte bestimmt, und diese werden punktweise auf das PET-Bild angewendet.

Ein Nachteil dieses Verfahrens ergibt sich aus der Strahlenbelastung für den untersuchten Organismus. Für die CT-Aufnahme wird der Organismus Röntgenstrahlen ausgesetzt, die in hohen Dosen verabreicht zu Hautverbrennungen, Zellmutation oder dergleichen führen können.

Ein weiteres Verfahren zum Durchführen einer Schwächungs- und Streukorrektur für ein PET-Bild ist aus der EP 1 105 750 B1 bekannt.

Das dort erläuterte Verfahren basiert auf einer so genannten "Atlasregistrierung" des aufgenommenen PET-Bildes. Ein Atlas ist als dreidimensionales Computermodell ausgebildet und beinhaltet zwei Komponenten, eine funktionelle Komponente und eine anatomische Komponente. Die funktionelle Komponente kann aus CT-, MR- oder PET-Bildern, und die anatomische Komponente kann aus Transmissionsaufnahmen (beispielsweise CT-Bildern) gebildet werden.

Das aufgenommene PET-Bild wird mit der funktionellen Komponente des Atlas "registriert", d.h. das PET-Bild wird durch Vergleichen, insbesondere unter Anwendung einer Transformation, mit der funktionellen Komponente des Atlasses (räumlich) ausgerichtet. Die hierzu auf das aufgenommene PET-Bild angewendete Transformation beinhaltet lineare Abbildungen (Rotation, Translation, Skalierung) sowie nicht-lineare Abbildungen zur Deformation des PET-Bildes. Die ermittelte Transformation wird auf die anatomische Komponente des Atlasses angewendet, um hieraus eine Schwächungskarte für das PET-Bild zu bestimmen.

Ein Nachteil dieses Verfahrens besteht darin, dass die funktionelle Komponente des Atlasses, insbesondere wenn sie durch CT-Bilder gebildet wird, einen schlechten Weichteilkontrast bietet. Die Registrierung des aufgenommenen PET-Bildes erfolgt dann anhand der Umrisslinien von Knochen, die aber wiederum im PET-Bild "verwischt" bzw. nur schwer erkennbar sind. Hierdurch ist die Ausrichtung des PET-Bildes im Atlas erschwert, so dass die ermittelte Transformation fehlerhaft und folglich die mittels dieser Transformation erzeugte Schwächungskarte unzureichend für eine Korrektur des PET-Bildes sein kann.

Ferner sind Verfahren für eine Schwächungs- und Streukorrektur von γ-Strahlung für ein PET-Bild bekannt, die basierend auf MR-Bildern durchgeführt werden.

Ein bekanntes Verfahren, ein PET-Bild basierend auf einem MR-Bild zu korrigieren, beruht darauf, für jeden interessierenden Punkt des PET-Bildes den bezüglich seiner räumlichen Lage entsprechenden Punkt im MR-Bild zu bestimmen. Danach wird für den entsprechenden Punkt des MR-Bildes direkt auf den Schwächungs- und Streuungswert geschlossen, der dann auf den interessierenden Punkt des PET-Bildes angewendet wird, um so eine rechnerische Korrektur des interessierenden Punkts zu ermöglichen.

Ein Punkt eines PET- oder eines MR-Bildes bezeichnet hierbei ein Pixel, eine Grundeinheit eines zweidimensionalen Bildes, oder ein Voxel, das dreidimensionale Analogon des Pixels.

Nachteilig an diesem bekannten Verfahren ist, dass die Zuordnung zwischen einer Punktintensität im MR-Bild und eines Korrekturwerts nicht eindeutig definiert ist. Der Punkt des MR-Bildes enthält nicht genügend Information, um allein aus seiner Intensität auf die ihm zugrunde liegende morphologische Struktur (Knochen, Gewebe usw.) zu schließen, die die Abschwächung der γ-Strahlung sowie auftretende Vielfachstreuprozesse bestimmt. Es ist daher beispielsweise möglich, dass einem Punkt, der einen Knochen abbildet, der Schwächungswert für Luft zugeordnet wird, da Luft und Knochen im MR-Bild nur schwer unterscheidbar sind.

Ein weiteres Verfahren zum Korrigieren eines PET-Bildes basierend auf einem MR-Bild berechnet aus dem MR-Bild eine primitive Schwächungskarte. Hierzu wird im MR-Bild zwischen nur zwei Helligkeitsstufen unterschieden, und diesen Helligkeitsbereichen wird jeweils ein Schwächungswert zugeordnet. Beispielsweise kann im MR-Bild zwischen innerhalb des Körpers liegenden Punkten und außerhalb des Körpers liegenden Punkten unterschieden werden. Ein Nachteil dieses Verfahrens beruht darauf, dass bei der Erstellung der Schwächungskarte die im MR-Bild enthaltenen Informationen bezüglich der Anatomie des Körpers nicht berücksichtigt werden, so dass die erstellte Schwächungskarte ungenau ist. Wendet man diese ungenaue Schwächungskarte zur Korrektur auf ein PET-Bild an, so ist das korrigierte PET-Bild ebenfalls unpräzise.

Ferner ist aus der US 2006/0058641 A1 ein Verfahren sowie eine Vorrichtung zum Bestimmen einer Schwächungskarte für ein PET-Bild basierend auf einem MR-Bild bekannt. Bei diesem Verfahren wird ein so genannter MR-Atlas verwendet, der als normiertes dreidimensionales Modell mit hinterlegten Schwächungskarten ausgebildet ist. Das aufgenommenes MR-Bild wird mittels einer Transformation in Form einer linearen Abbildung (Rotation, Translation, Skalierung) mit dem MR-Atlas registriert, so dass durch Vergleichen die räumliche Lage des MR-Bildes innerhalb des MR-Atlasses bestimmt wird. Hierbei wird das aufgenommene MR-Bild durch die ermittelte Transformation auf das normierte dreidimensionale Modell angepasst. Diese Transformation wird auf die zugehörige Schwächungskarte angewendet, so dass sich eine Schwächungkarte für das aufgenommene PET-Bild ergibt.

Dieses Verfahren erweist sich als besonders nachteilig, wenn der aufgenommene Körperabschnitt untypische Anomalien, beispielsweise von der Norm abweichende Armlängen, Organgrößen oder dergleichen, aufweist, da diese im MR-Atlas, der aus Bildern von Referenzpersonen gebildet ist, nicht vorhanden sind. Hierdurch ist eine Registrierung des MR-Bildes mit dem MR-Atlas erschwert. Oft kommt es zu stark fehlerhaften Registrierungen Wendet man diese unzureichende Transformation auf die hinterlegten Schwächungskarten des MR-Atlasses an, so ergibt sich eine fehlerhafte Schwächungskarte für das unkorrigierte PET-Bild.

Der Aufsatz von Habib Zaidi et al: " Magnetic resonance imaging-guided attenuation and scatter corrections in three-dimensional brain positron emission tomography", in Med Physics, May 2003, Volume 30(5), Seiten 937-48, offenbart die Grundidee der Segmentierung (auf Basis einer FCM-Clusterung) eines MR-Bildes zur Ermittlung der Schwächungskarte für ein PET-Bild.

Es besteht daher weiterhin ein Bedürfnis nach einem verbesserten Verfahren zum Bestimmen einer Eigenschaftskarte, insbesondere einer Schwächungskarte, für einen Gegenstand basierend auf zumindest einem ersten Bild, insbesondere einem MR-Bild, das auf sehr einfache Weise, mit hoher Genauigkeit und zugleich fehlerfrei die Eigenschaftskarte vorhersagt.

Erfindungsgemäß wird die Aufgabe hinsichtlich des eingangs genannten Verfahrens gelöst, indem eine Struktur für Referenzpaare definiert wird, wobei jedes Referenzpaar zumindest zwei Einträge aufweist, wobei der erste Eintrag einen Eigenschaftswert, insbesondere einen Schwächungswert, darstellt, und wobei der zweite Eintrag eine Gruppe von zusammengehörenden Bildpunkten, die insbesondere aus MR-Bildern extrahiert sind, darstellt, die zumindest einen dem Eigenschaftswert entsprechenden interessierenden Bildpunkt umfasst, indem ferner eine Vielzahl von Trainingspaaren bereitgestellt wird, wobei eine Struktur der Trainingspaare der Struktur der Referenzpaare entspricht, und wobei die Einträge der jeweiligen Trainingspaare bekannt sind, und indem ferner eine Zuordnung zwischen den ersten Einträgen und den weiteren Einträgen der Trainingspaare durch maschinelles Lernen bestimmt wird, um so für einen beliebigen Punkt der ersten Bildes einen entsprechenden Eigenschaftswert vorhersagen zu können.

Bei dem erfindungsgemäßen Verfahren wird ein neuer Ansatz zum Bestimmen der Eigenschaftskarte für einen Gegenstand basierend auf einem zumindest ersten Bild gewählt. Erfindungsgemäß wird die Eigenschaftskarte, insbesondere die Schwächungskarte, mittels Methoden des maschinellen Lernens, insbesondere durch Klassifikation und Regression, bestimmt. Mit einer Support-Vektor-Maschine wird z.B. anhand von Trainingsdaten eine Zuordnung zwischen bekannten Eigenschaftswerten (d.h. einem ersten Eintrag) und weiteren bekannten Informationen in Form von zweiten und/oder weiteren Einträgen bestimmt. Die einmal gelernte Zuordnung kann dann auf beliebige Punkte des Gegenstands angewendet werden, dessen Eigenschaftswerte unbekannt sind, um die entsprechenden Eigenschaftswerte vorherzusagen. Hierdurch kann die Vorhersage vorteilhafterweise besonders schnell durchgeführt werden, da nur auf das bereits gelernte Wissen zurückgegriffen werden muss und nicht weitere Schritte beim Ermitteln der unbekannten Eigenschaftswerte durchgeführt werden müssen.

Ein weiterer Vorteil des erfindungsgemäßen Verfahrens besteht darin, dass das Bestimmen der Eigenschaftskarte, abhängig von der Anzahl der Trainingsbeispiele und den beim maschinellen Lernen berücksichtigten Informationen, sehr genau durchgeführt werden kann. Insbesondere ist die Bestimmung der Eigenschaftskarte für ein bestimmtes Bild reproduzierbar.

In einer bevorzugten Ausgestaltung weist die Struktur der Referenzpaare zusätzlich einen dritten Eintrag auf, der beim Bestimmen der Zuordnung zum ersten Eintrag zusammen mit dem zweiten Eintrag berücksichtigt wird.

Diese Maßnahme hat den Vorteil, dass eine weitere (Quelle der) Information bereitgestellt wird, die beim maschinellem Lernen berücksichtigt wird. Hierdurch verbessert sich die Genauigkeit der zu lernenden Zuordnungsvorschrift signifikant, da diese komplexere Zusammenhänge zwischen den Einträgen erkennen und verarbeiten lernen kann.

In einer weiteren bevorzugten Ausgestaltung stellt der dritte Eintrag dar: eine registrierte Koordinate; eine weitere Gruppe von zusammengehörenden Bildpunkten eines zumindest zweiten Bildes, wobei die weitere Gruppe von Bildpunkten des zumindest zweiten Bildes zumindest teilweise einen gleichen Gegenstandsausschnitt wie die Gruppe der Bildpunkte des ersten Bildes zeigt; und/oder eine Klassenzugehörigkeit.

Die Maßnahme stellt vorteilhafterweise viele verschiedene Informationen für den dritten Eintrag der Struktur der Referenzpaare bereit, die einzeln oder auch kombiniert, je nach gewünschter Zielsetzung für die Zuordnung, beim Lernen und Anwenden der Zuordnung verwendet werden, um die Zuordnungsgenauigkeit zu verbessern. Eine "registrierte Koordinate" erhält man dadurch, dass das zumindest erste (vorgegebene) Bild mit einem Referenzbild registriert wird. Dazu wird eine lineare (Trabslation, Rotation , Skalierung) und/oder eine nicht-lineare Abbildung (Deformation) bestimmt, die jedem Punkt im gegebenen Bild einen Punkt im Referenzbild zuordnet. Die "registrierte Koordinate" eines Punktes im gegebenen Bild ist die Koordinate des zugeordneten Bildpunkts im Referenzbild. Sie wird relativ zu einem vom Referenzbild abhängigen Koordinatensystem angegeben. Dabei handelt es sich bei diesem Koordinatensystem des Referenzsystems nicht notwendig um ein kartesisches Koordinatensystem. Insbesondere ist eine inhomogene Dichte der Gitterlinien des Koordinaten-systems denkbar, wobei die Dichte ortsabhängig von bestimmten Eigenschaften des Referenzbildes an diesem Ort bestimmt werden kann.

In einer besonderen Ausgestaltung können auch mehrere Referenzbilder verwendet werden. Die Registrierung erfolgt mittels bekannter Algorithmen (siehe z.B. "A survey of medical image registration", J.B. Antoine Maintz und Max Viergever, Medical Image Analysis (1998), Ausgabe 2, Nummer 1, S. 1 36). Alternativ kann die Registrierung auch rein oberflächenbasiert erfolgen, wie z.B. in B. Schölkopf, F. Steinke, und V. Blanz, "Object correspondance as a machine learing problem", Proceedings of the 22nd International Conference on Machine Learing, 777 - 784, (Eds.) L. De Raedt, S. Wrobel, ACM Press, 2005 offenbart. Auch bei kleinen Fehlern in der Registrierung, wie sie üblicherweise vorkommen, kann die registrierte Koordinate als zusätzliche Information die Genauigkeit der Vorhersage signifikant erhöhen.

In einer weiteren bevorzugten Ausgestaltung werden benötigte Abstände zwischen registrierten Koordinaten mittels eines vom Ort im Referenzgegenstand abhängigen Abstandsmaß bestimmt, und nicht wie üblich mit einem euklidischen Abstand.

Viele Methoden des maschinellen Lernens bestimmen implizit die Ähnlichkeit zwischen verschiedenen Eingabeeinträgen. Die Verwendung eines ortsabhängigen Abstandsmaßes hat den Vorteil, dass die Gewichtung der registrierten Koordinaten im verwendeten Ähnlichkeitsmaß ortsabhängig unterschiedlich eingestellt werden kann. Somit legt die Vorhersage in Regionen, in denen die Registrierung typischerweise fehlerfrei verläuft (z.B. im Schädel) mehr Wert auf die registrierten Koordinaten als in Regionen, in denen öfter Fehler auftreten (z.B. im Bauch).

In einer weiteren bevorzugten Ausführung geben die registrierten Koordinaten die Abstände des zum interessierenden Bildpunkt korrespondierenden Punktes im Referenzbild zu bestimmten anatomischen charakteristischen Punkten.

Dies hat den Vorteil , dass die charakteristischen Punkte zuverlässig gefunden werden können. Insgesamt erhöht sich die Genauigkeit der Vorhersage.

Die Verwendung verschiedener Gruppen von Bildpunkten mit verschiedener Aufnahmemodalität erhöht vorteilhafterweise die Genauigkeit beim Lernen der Zuordnung, so dass folglich das Vorhersagen eines unbekannten Eigenschaftswerts verbessert wird.

Die Verwendung einer Klassenzugehörigkeit ermöglicht das Lernen der Zuordnung nicht nur basierend auf Eigenschaften der Gruppen von Bildpunkten, sondern es werden zusätzlich Informationen über Eigenschaften des untersuchten Gegenstands berücksichtigt.

In einer weiteren bevorzugten Ausgestaltung ist das zumindest zweite Bild ein Positronenemissionstomographiebild, PET-Bild, oder ein weiteres MR-Bild. Insbesondere können bei Verwendung von einem oder mehreren PET-Bildern ein Zeitpunkt der PET-Aufnahme nach einer Tracer-Injektion sowie Eigenschaften und Menge des Tracers als weiterer Eintrag in der Struktur verwendet werden.

Die Maßnahme hat den Vorteil, dass insbesondere bei der Bestimmung einer Schwächungskarte für ein PET-Bild basierend auf einem MR-Bild die Referenzpaare zusätzlich zu den Gruppen von Bildpunkten aus MR-Bildern weitere Gruppen von Bildpunkten aufweisen, die die gleiche Aufnahmemodalität wie die beiden bereitgestellten Bilder aufweisen. Somit müssen nicht zusätzliche Bilder anderer Aufnahmemodalität oder Informationen mittels anderer Untersuchungsmethoden bereitgestellt werden. Insbesondere können die MR-Bilder eine T₁- oder T₂-Gewichtung aufweisen, so dass die kombinierte Information als Eingabe beim maschinellen Lernen berücksichtigt werden kann.

In einer weiteren bevorzugten Ausgestaltung umfasst die Klassenzugehörigkeit: ein Patientenalter, ein Patientengeschlecht, ein Patientengewicht und/oder eine Patientenlebensgewohnheit.

Die Maßnahme hat den Vorteil, dass beim Lernen und Anwenden einer Zuordnung im Bereich der medizinischen Bildverarbeitung Informationen über Patienten berücksichtigt werden können.

In einer weiteren bevorzugten Ausgestaltung definiert die Gruppe von zusammengehörenden Bildpunkten ein vorzugsweise rechtwinkliges Fenster.

Die Maßnahme hat den Vorteil, dass die Gruppe von Bildpunkten ein zusammenhängendes Gebiet bildet, das auf einfache Weise ausgewählt werden kann und dessen Eigenschaften beim maschinellen Lernen berücksichtigt werden können. Die Abmessung des Fensters kann, je nach Zielsetzung, frei oder rechnergestützt durch ein Computerprogramm zur "Modell-Selektion" gewählt werden.

In einer weiteren bevorzugten Ausgestaltung wird aus der Gruppe von zusammengehörenden Bildpunkten eine Untergruppe ausgewählt, die den interessierenden Bildpunkt aufweist.

Die Maßnahme hat den Vorteil, dass nicht nur Flächeneigenschaften der Gruppe, sondern auch solche, die sich aus einer Linie oder auch aus Ringen/Kugelschalen um den interessierenden Punkt ergeben, beim Lernen berücksichtigt werden.

In einer weiteren bevorzugten Ausgestaltung weist jeder Bildpunkt einen Intensitätswert auf.

Die Maßnahme hat den Vorteil, dass der Intensitätswert eine sehr einfache Information darstellt, die beim maschinellen Lernern berücksichtigt wird. Der Intensitätswert eines Bildpunkts kann bspw. im Falle eines MR-Bildes eine Helligkeitsstufe/ein Grauwert sein, die/der einfach aus dem MR-Bild ermittelt werden kann.

In einer weiteren bevorzugten Ausgestaltung wird zu jeder Gruppe von zusammengehörenden Bildpunkten eine Textur bestimmt.

Unter einer "Textur" der Gruppe von Bildpunkten ist bspw. eine Intensitätsverteilung (Helligkeitsstufen/Grauwerte) der Bildpunkte zu verstehen, aus der - je nach Größe der Gruppe - Umrisslinien oder dgl. erkennbar sein können. Das maschinelle Lernen der Zuordnung sowie deren Anwenden basierend auf der Textur der Gruppe stellt vorteilhafterweise eine weitere Information bereit, die bei dem erfindungsgemäßen Verfahren berücksichtigt werden kann.

In einer weiteren bevorzugten Ausgestaltung wird basierend auf der Untergruppe ein Intensitätshistogramm erstellt, das der Struktur als weiterer Eintrag hinzugefügt werden kann.

Die Maßnahme hat den Vorteil, dass eine weitere (Quelle der) Information für das maschinelle Lernen bereitgestellt wird. Das Intensitätshistogramm gibt die Intensitätsverteilung der Bildpunkte bzgl. der Untergruppe an.

In einer weiteren bevorzugten Ausgestaltung wird basierend auf den Intensitätswerten zumindest der Bildpunkte der Gruppe von zusammengehörenden Bildpunkten ein Intensitätsmittelwert gebildet, der zusammen mit der Intensität des interessierenden Bildpunkts als weiterer Eintrag der Struktur hinzugefügt werden kann.

Die Maßnahme hat den Vorteil, dass eine noch weitere (Quelle der) Information für das maschinelle Lernen bereitgestellt wird, um die Zuordnungsgenauigkeit zu erhöhen. Die Verwendung des Intensitätsmittelwerts der Gruppe von Bildpunkten oder des gesamten Bildes relativ zu dem Intensitätswert des interessierenden Bildpunkts kann bspw. bildabhängige Eigenschaften, die durch die Bildaufnahme bedingt sind, berücksichtigen.

In einer weiteren bevorzugten Ausgestaltung werden das zumindest erste Bild, die MR-Bilder und/oder das zumindest zweite Bild insbesondere durch eine Wavelet-Tranformation, Fourier-Transformation SIFT, Kantendetektoren oder Kontrastverstärkung vorverarbeitet.

Die Maßnahme hat den Vorteil, dass Prozessschritte zur Merkmals-Extraktion auf das zumindest erste Bild, die MR-Bilder, das zumindest zweite Bild oder auch auf die Gruppen von Bildpunkten, die aus diesen Bildern gebildet sind, angewendet werden. Mittels dieser Methoden können bspw. der Bildkontrast verstärkt und einzelne Bildmerkmale besser herausgearbeitet werden.

In einer weiteren bevorzugten Ausgestaltung werden die zweiten und weiteren Einträge der Datenstrukturen einem Dimensionalitätsreduktionsverfahren, insbesondere einer Hauptkomponenten analyse oder einem Codebook, zugeführt, dessen neu gewonnene Datenbeschreibung der Datenstruktur als weiterer Eintrag hinzugefügt und zur Vorhersage mitverwendet wird.

Dies hat den Vorteil, dass die Einträge so kompakt zusammengefasst werden und für dieses Problem charakteristische Merkmale hervorgehoben werden. Insgesamt kann so die Geschwindigkeit der Vorhersage der Eigenschaftskarte erhöht werden.

In einer weiteren bevorzugten Ausgestaltung wird für den beliebigen Punkt des Gegenstands eine Struktur, die der Struktur der Referenzpaare entspricht, bereitgestellt, und die Struktur des beliebigen Punkts weist zumindest einen von dem ersten Eintrag verschiedenen, bekannten Eintrag auf, wobei der erste Eintrag fehlt.

Die Maßnahme hat den Vorteil, dass für den beliebigen Punkt, dessen Eigenschaftswert unbekannt ist, die gleiche Struktur wie für die Referenzpaare bereitgestellt wird, so dass die Struktur des beliebigen Punkts als Eingabe für das maschinelle Lernen verwendet werden kann.

In einer weiteren bevorzugten Ausgestaltung erfolgt das Vorhersagen des ersten Eintrags des beliebigen Punkts durch Anwenden der gelernten Zuordnung basierend auf zumindest einem oder mehreren der bekannten Einträge des beliebigen Punkts.

Die Maßnahme ermöglicht vorteilhafterweise eine schnelle und genaue Vorhersage des unbekannten Eigenschaftswerts, d.h. des ersten Eintrags, des beliebigen Punkts basierend auf einem oder mehreren bekannten Einträgen.

In einer weiteren bevorzugten Ausgestaltung erfolgt die Vorhersage im Wesentlichen basierend auf den zweiten Einträgen.

Die Maßnahme hat den Vorteil, dass die Gruppe der Bildpunkte des beliebigen Punkts sowie die aus ihr bestimmten Eigenschaften eine wichtige Informationsquelle beim Vorhersagen des unbekannten Eigenschaftswerts des beliebigen Punkts darstellen.

In einer weiteren bevorzugten Ausgestaltung erfolgt die Vorhersage, wenn für den beliebigen Punkt kein zweiter Eintrag bekannt ist, basierend auf einem oder mehreren bekannten Einträgen.

Die Maßnahme hat den Vorteil, dass auch, wenn die Information über die Gruppe der Bildpunkte fehlt, der Eigenschaftswert genau und schnell vorausgesagt werden kann, da die weiteren bekannten Einträge eine zusätzliche vom zweiten Eintrag unabhängige Informationsquelle darstellen.

In einer weiteren bevorzugten Ausgestaltung erfolgt die Vorhersage zusätzlich, wenn der dritte Eintrag der Struktur eine registrierte Koordinate darstellt, basierend auf der registrierten Koordinate.

Die Maßnahme hat den Vorteil, dass eine kombinierte Information des zweiten Eintrags und des dritten Eintrags in Form der registrierten Koordinate eine genaue und zuverlässige Vorhersage des unbekannten Eigenschaftswerts ermöglicht.

In einer weiteren bevorzugten Ausgestaltung wird zusätzlich das zumindest zweite Bild zur Registrierung mit dem zumindest ersten Bild und zum Erweitern des zumindest ersten Bildes verwendet.

Diese Maßnahme hat den Vorteil, dass noch eine weitere (Quelle der) Information zum Vorhersagen des ersten Eintrags des beliebigen Punkts des zumindest ersten Bildes verwendet werden kann. Diese Information wird durch das bereits aufgenommene zweite Bild bereitgestellt, so dass weder weitere Bilder noch andere Informationen durch andere Methoden bereitgestellt werden müssen.

Die Erweiterung des zumindest ersten Bildes ist insbesondere vorteilhaft, wenn das zumindest erste Bild einen kleineren Ausschnitt als das zumindest zweite Bild zeigt.

Dies ist bspw. bei einer MR-Brustaufnahme eines Menschen der Fall, da das zusätzlich aufgenommene PET-Bild mit angelegten Armen, das MR-Bild hingegen mit in axialer Richtung über den Kopf gestreckten Armen aufgenommen wird. Obwohl das MR-Bild einen kleineren Ausschnitt zeigt, kann dann die Eigenschaftskarte, d.h. die Schwächungskarte, für das gesamte PET-Bild bereitgestellt werden. Ferner können hierdurch Aufnahme-Artefakte im MR-Bild ausgeglichen werden, da basierend auf einer Registrierung mit dem PET-Bild der fehlerhafte Bereich des MR-Bildes erweitert werden kann.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele im Zusammenhang mit der beiliegenden Zeichnung näher beschrieben und erläutert. Es zeigen:
- Fig. 1: eine schematische Darstellung eines Trainingsbeispiels für eine Lernma- schine mit einem MR-Bild und einer entsprechenden Schwächungskarte;
- Fig. 2: eine schematische Darstellung einer Eingabe für die Lernmaschine;
- Fig. 3: eine schematische Darstellung eines PET-Bildes, eines entsprechenden MR-Bildes und einer zugehörigen Schwächungskarte;
- Fig. 4A: einen Ausschnitt des MR-Bildes in Fig. 1;
- Fig. 4B: eine Schwächungskarte für ein PET-Bild, das den Ausschnitt in Fig. 4A zeigt und mit dem erfindungsgemäßen Verfahren (ohne registrierte Koor- dinaten) berechnet wurde;
- Fig. 4C: eine weitere Schwächungskarte für ein PET-Bild, das den Ausschnitt in Fig. 4A zeigt und mit dem erfindungsgemäßen Verfahren (mit registrierte Koordinaten) berechnet wurde; und
- Fig. 4D: eine Schwächungskarte für ein PET-Bild, das den Ausschnitt in Fig. 4A zeigt; und
- Fig. 5: ein Flussdiagramm eines erfindungsgemäßen Verfahrens;

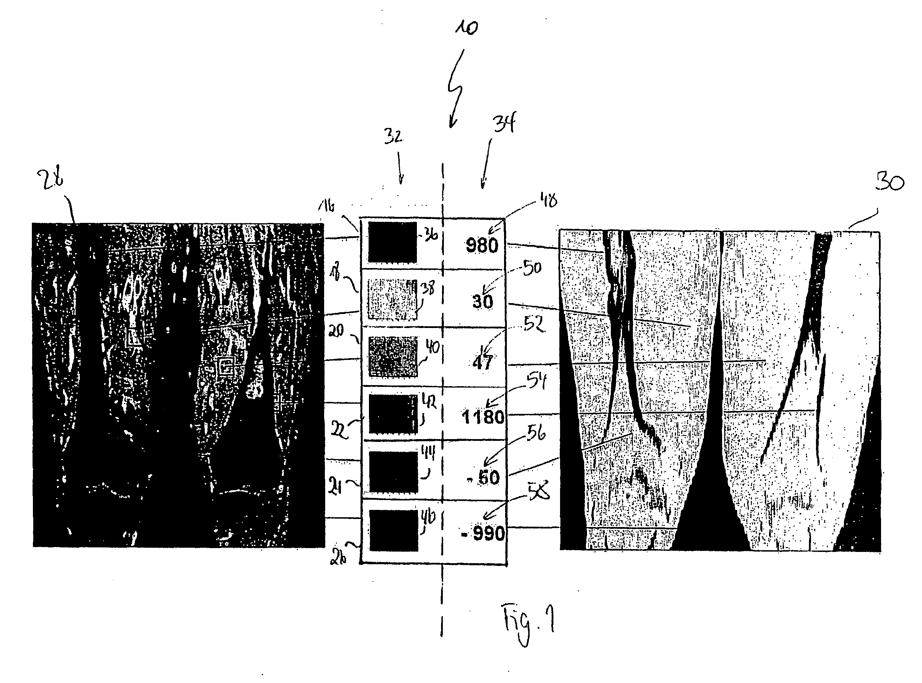

In Fig. 1 ist ein mit dem allgemeinen Bezugszeichen 10 versehenes Trainingsbeispiel dargestellt, das bei einem Verfahren 12 zum Bestimmen einer Eigenschaftskarte für einen Gegenstand basierend auf zumindest einem ersten Bild verwendet wird.

Das Verfahren wird bspw. in der medizinischen Bildverarbeitung zum Bestimmen einer Schwächungskarte zur Korrektur eines PET-Bildes eines Lebewesens basierend auf einem MR-Bild verwendet. Mittels des Verfahrens sollen zwei Aufnahmeeffekte des PET-Bildes, Abschwächung sowie Streuung einer bei einer Positron-Elektron-Annihilation auftretende Gammastrahlung, quantitativ zumindest teilweise rechnerisch korrigiert werden.

Im Allgemeinen wird das Verfahren der Erfindung verwendet, um für einen Gegenstand eine Eigenschaftskarte zu bestimmen. Alternativ können neben einem Eigenschaftswert für jeden interessierenden Punkt auch Wahrscheinlichkeiten für den Eigenschaftswert berechnet werden, und zwar für alle Punkte. Bei dem Verfahren der Erfindung werden Methoden des maschinellen Lernens verwendet, um zuerst - punktweise - für verschiedene Bildpunkte eines Bildes eine Zuordnung zwischen einem Eigenschaftswert (Ausgabe) und verschiedenen Informationen (Eingaben) zu lernen, d.h. es wird eine Zuordnungsvorschrift von der bzw. den Eingaben auf die Ausgabe gelernt. Später wird die gelernte Zuordnung auf einen beliebigen Punkt eines unbekannten, d.h. nicht gelernten, (Patienten-)Bildes angewendet, um basierend auf bekannten, d.h. den Eingaben entsprechenden, Informationen dieses Punktes dessen unbekannten Eigenschaftswert zu bestimmen.

Beispielsweise wird eine Zuordnung zwischen MR-Bildern und einem Schwächungswert gelernt, wobei der Schwächungswert durch ein einem MR-Bild entsprechendes CT-Bild repräsentiert sein kann. Wenn die Zuordnung "MR-Bild zu CT-Bild" gelernt ist, kann basierend auf einem MR-Bild eines Patienten ein CT-Bild berechnet werden, obwohl kein CT-Bild aufgenommen wurde. Diese Information kann dann wieder benutzt werden, um ein PET-Bild auf Schwächung zu korrigieren. Alternativ könnten auch andere Eigenschaften gelernt werden, wie z.B. eine Unterscheidung zwischen Tumorgewebe und gesundem Gewebe, eine Identifikation von in dem Bild enthaltenen Organen, Gewebe und Knochen, eine Unterscheidung zwischen Orten innerhalb und außerhalb des Patientenkörpers, und Ähnliches. Solche Eigenschaft werden auch als "Labels" bezeichnet.

Gemäß der vorliegenden Erfindung wird zunächst eine Struktur für Referenzpaare definiert. Jedes Referenzpaar weist dabei zumindest zwei Einträge auf. Ein erster Eintrag jedes Referenzpaars stellt den Eigenschaftswert (Label), d.h. die Ausgabe, dar, und die weiteren Einträge stellen die Eingaben (Informationsquellen) für das maschinelle Lernen dar. Insbesondere stellt der zweite Eintrag eine Gruppe ("Patch") von zusammengehörenden Bildpunkten dar, die einen interessierenden Bildpunkt umfasst. Der interessierende Bildpunkt ist der Punkt, den man sich herausgreift, um seine Eigenschaften zu bestimmen. Im einfachsten Fall wird die Gruppe von Bildpunkten durch ein Fenster repräsentiert, welches den interessierenden Punkt vorzugsweise in seiner (geometrischen) Mitte hat. Der zweite Eintrag wird vorzugsweise aus Bildern gleicher Aufnahmemodalität (z.B. gleiches bildgebendes Verfahren, gleiches Gesichtsfeld, usw.) wie das später zu bearbeitende Patienten-Bild bereitgestellt.

Ferner wird eine Vielzahl von Trainingspaaren, d.h. Bilder mit bekannten Eigenschaften, bereitgestellt, deren Struktur der Struktur der Referenzpaare entspricht. Eine Aufnahmemodalität der Bilder, aus denen die Trainingspaare vorzugsweise extrahiert werden, entspricht der Aufnahmemodalität des ersten Bildes. Die Einträge der Trainingspaare sind bekannt, so dass mittels maschinellen Lernens eine Zuordnung zwischen der Eingabe und der Ausgabe der Trainingspaare gelernt werden kann. Nachdem die Zuordnung gelernt ist, werden zumindest einige der Trainingspaare zu tatsächlichen Referenzpaaren, was aufgrund der gleichen Struktur kein Problem ist. Ferner kann die gelernte Zuordnung auf einen beliebigen Bildpunkt eines Patienten-Bildes angewendet werden, von dem einige Informationen, wie z.B. seine Intensität, bekannt sind, dessen Ausgabe, d.h. dessen Eigenschaftswert, aber unbekannt ist. Hierbei sollte die Struktur des beliebigen Punktes der Struktur der Referenzpaare entsprechen, d.h. wenn bei den Referenzpaaren ein Schwächungswert für eine Gruppe von Punkten (Patch) gelernt wurde, dann sollte der (unbekannte) interessierende Punkt in eine entsprechende Gruppe eingebettet werden, um die gelernte Zuordnung auf den unbekannten Punkt anwenden zu können. Das maschinelle Lernen beruht allgemein auf einer Mustererkennung und wird mit einer Lernmaschine, vorzugsweise mit einer Support-Vektor-Maschine, wie sie bspw. in B. Schölkopf und A.J. Smola, "Learning with Kernels", MIT Press, Cambridge, Massachusetts, 2002 beschrieben ist, durchgeführt. Das maschinelle Lernen stellt einen komplexen Vorgang dar und umfasst alternativ auch Support Vektor Regression, Gauß-Prozesse, k-Nearest-Neighbour-Verfahren, Regressions-/ Klassifikationsbäume oder dgl.

Sehr vereinfachend und bildhaft gesprochen, beruht das Vorhersagen des unbekannten Eigenschaftswerts (bzw. die Anwendung der gelernten Zuordnung auf) eines beliebigen Punkts im Patienten-Bild auf einem Vergleichen der Eingabe des beliebigen Bildpunkts mit den Eingaben der Referenzpaare, so dass z.B. eine bestübereinstimmende Eingabe aus den Eingaben der Referenzpaare ausgewählt wird. Dem beliebigen Punkt kann dann der Eigenschaftswert des Referenzpaares zugeordnet werden, der diese bestübereinstimmende Eingabe aufweist. Die tatsächliche Vorhersage ist natürlich bedeutend komplexer, nämlich genauso komplex wie das maschinelle Lernen selbst. Der Eigenschaftswert wird tatsächlich berechnet. Jedoch erscheint das Bild eines Vergleichens mit einer Liste von Referenzpaaren mit anschließendem Auswählen eines besten Paars recht anschaulich zu sein. Nachfolgend angegebene Beispiele dienen also lediglich der Vereinfachung einer Veranschaulichung und sind deshalb keinesfalls einschränkend aufzufassen.

Zurückkehrend zum erfindungsgemäßen Verfahren können Gruppen von Bildpunkten der verwendeten Bilder, d.h. einzelne Teilbereiche oder auch die gesamten Bilder, durch Bildverarbeitungsschritte, wie Fourier-Transformation, SIFT, Kantendetektoren, Kontrastverstärkung oder dgl. vorverarbeitet werden, um die Musterextraktion zu verbessern. Die gilt sowohl beim Lernen aus bekannten Bildern als auch bei der späteren Anwendung auf unbekannte Bilder.

Der zweite Eintrag, d.h. die Gruppe von zusammengehörenden Bildpunkten, definiert ein vorzugsweise rechtwinkliges Fenster, das den interessierenden Bildpunkt, für den der Eigenschaftswert gelernt werden soll, umfasst. Das Fenster könnte jedoch auch kreisförmig sein oder eine beliebig definierte Umrandung aufweisen. Vorzugsweise umfasst das Fenster neben dem interessierenden Punkt zumindest einen weitern Punkt, insbesondere einen direkten Nachbarn. Allgemein muss die Gruppe aus zusammengehörigen Punkten jedoch nicht aus zusammenhängenden Punkten bestehen. Eine Abmessung des Fensters ist je nach gewünschter Zielsetzung manuell wählbar. Sie kann mittels eines Computerprogramms zur Modell-Selektion ausgewählt werden. Die Fensterabmessung kann die Genauigkeit und die Rechenzeit des Verfahrens der Erfindung beeinflussen. Sind bspw. die Bilder durch rasterförmig angeordnete Punkte, Pixel (zweidimensionale Grundeinheit eines zweidimensionalen Bildes) oder Voxel (dreidimensionale Grundeinheit eines dreidimensionalen Bildes), definiert, kann das Fenster im Falle von Pixeln quadratisch oder rechteckig und im Falle von Voxeln würfel- oder quaderförmig ausgebildet sein. Eine Seitenlänge des Fensters kann Werte im Bereich von Millimetern oder Zentimetern aufweisen, so dass die Gruppe von Bildpunkten ebenfalls ein vollständiges Bild oder einen Ausschnitt des Bildes darstellen kann. Ferner ist es möglich, dass der interessierende Punkt mittig im Fenster angeordnet ist oder aus einem Mittelpunkt des Fensters verschoben ist. Die Abmessung wird vorzugsweise in der Größenordnung von Bildpunkten definiert.

Bezug nehmend auf Fig. 1 ist im linken Bereich der Figur exemplarisch ein MR-Bild von Oberschenkeln, in der Mitte eine "Liste" von Referenzpaaren bzw. Trainingspaaren und im rechten Bereich eine Schwächungskarte gezeigt, die dem linken Bild entspricht. Das in Fig. 1 gezeigte Trainingsbeispiel 10 für die Lernmaschine weist sechs Trainingspaare 16 - 26 auf. Das Trainingsbeispiel 10 weist ein MR-Bild 28 und die zu dem MR-Bild 28 entsprechende Schwächungskarte 30 auf, die hier exemplarisch ein CT-Bild ist. Die Bilder 28 und 30 wurden von der gleichen Person aufgenommen, vorzugsweise ohne Versatz der Peron zwischen den Aufnahmen und vorzugsweise bei gleichem Gesichtfeld ("Field of View" ).

Mittels des Trainingsbeispiels 10 wird ein Zuordnung von einer Eingabe 32 auf eine Ausgabe 34 gelernt. In der Fig. 1 sind jeweils die Eingaben links und die Ausgaben rechts bei den Trainingspaaren 16 - 26 gezeigt, die zu Veranschaulichungszwecken durch eine gestrichelte Linie voneinander getrennt dargestellt sind. Die Eingaben 32 stellen jeweils den zweiten Eintrag der Struktur der Trainingspaare 16 - 26 in Form von sechs, hier rechtwinkligen, Fenstern 36 - 46 dar, die aus dem MR-Bild 28 extrahiert sind. Ferner werden Schwächungswerte 48 - 58 bereitgestellt, die man z.B. aus dem CT-Bild 30 extrahiert.

Dies bedeutet detaillierter, dass man sich Punkte im MR-Bild 28 auswählt, ein Fenster um die ausgewählten Punkt legt, diese Fenster "ausschneidet" und ihnen einen Schwächungswert zuweist. Den Schwächungswert erhält man, indem man im CT-Bild 30 (geometrisch) den Punkt sucht, der dem im MR-Bild 28 ausgewählten Punkt entspricht, und dann den Schwächungswert des im CT-Bild 30 derart aufgefundenen Punktes extrahiert. Auf diese Weise lassen sich eine Vielzahl von Trainingspaaren 16 - 26 bilden, die für ein anschließendes Bestimmen einer Zuordnung zwischen den Eingaben 32 und den Ausgaben 34 erforderlich sind.

Eine Zuordnung von den Fenstern 36 - 46 zu den zugehörigen, bekannten Schwächungswerten 48 - 58 wird mittels maschinellen Lernens bestimmt. Jeweils ein Fenster 36 - 46 und ein Schwächungswert 48 - 58 bilden die Trainingspaare 16 - 26, die später dann als Referenzpaare 16 - 26 zur Vorhersage eines unbekannten Schwächungswerts eines neuen Fensters verwendet werden. In diesem Sinne sind Trainingspaare und Referenzpaare gleich. In der Praxis wird die gelernte Zuordnung dann auf einen unbekannten Punkt in einem Bild angewendet, um durch Berechnung seinen zugehörigen (unbekannten) Eigenschaftswert zu bestimmen. Ein tatsächlicher Vergleich mit den Referenzpaaren erfolgt üblicherweise nicht. Insofern sind die Begriffe "Trainingspaar" und "Referenzpaar" synonym und werden nur zur Vereinfachung einer Erläuterung und eines Verständnisses verwendet.

Beim (maschinellen) Lernen der Zuordnung von der Eingabe 32 auf die Ausgabe 34 können charakteristische Merkmale der Fenster 36 - 46 verwendet werden, wie z.B. eine Intensitätsverteilung. In dem MR-Bild 28 entspricht die Intensität eines Bildpunkts einer Helligkeitsstufe/einem Grauwert. Das Fenster 38 zeigt einen rechtwinkligen Bildausschnitt des rechten Oberschenkels der Person, in welchem ein Oberschenkelmuskel enthalten ist. Die Intensitätsverteilung im Fenster 38 lässt einen diagonal von links oben nach rechts unten verlaufenden helleren Teilbereich erkennen, der beim Lernen der Zuordnung auf den entsprechenden Schwächungswert 50 berücksichtigt wird. Ferner zeigt das Fenster 42 den Oberschenkelmuskel und einen Oberschenkelknochen, so dass die Intensitätsverteilung des Fensters 42 zwei vertikal voneinander getrennte Bereiche aufweist, von denen ein erster Bereich hell und ein zweiter Bereich dunkel ausgebildet ist. Der helle Bereich zeigt den Muskel, und der dunkle Bereich den Knochen. Die Fenstergröße ist hier derart bemessen, dass sowohl der Knochen als auch der an ihn angrenzende Muskel zu erkennen ist, so dass dieses Merkmal beim Lernen und beim Anwenden der Zuordnung berücksichtigt und ein fehlerhaftes Vorhersagen eines unbekannten Schwächungswert weitgehend ausgeschlossen werden kann. Würde das Fenster 42 bspw. nur den dunklen Bereich aufweisen, so könnte einem Fenster, das dem Fenster 42 ähnelt, irrtümlich einem Schwächungswert von Luft zugeordnet werden, da Luft üblicherweise eine sehr dunkle Intensitätsverteilung besitzt. Deshalb verwendet man vorzugsweise "charakteristische" Paare, d.h. Paare, in denen Texturen und Ähnliches deutlich erkennbar sind.

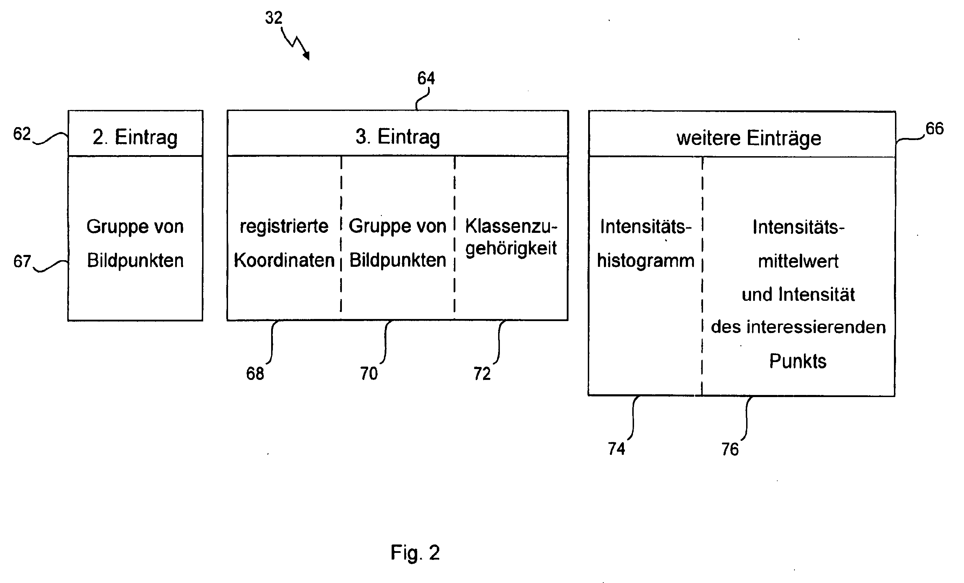

In Fig. 2 sind exemplarisch Einträge (Informationsquellen) 62 - 66 dargestellt, die als Eingaben 32 verwendbar sind und die beim Lernen der Zuordnung berücksichtigt werden können. Die einzelnen Einträge 62 - 66 können unabhängig voneinander bereitgestellt werden und sind gleich bedeutsam für das maschinelle Lernen. Der zweite Eintrag 62 umfasst eine Gruppe 67 von zusammengehörenden Bildpunkten, die einen eigentlich interessierenden Bildpunkt aufweisen. Wie zuvor erläutert, definiert die Gruppe von Bildpunkten das Fenster.

Jeder Bildpunkt weist hier eine Intensität auf, so dass zu jeder Gruppe von Bildpunkten eine "Textur" bestimmt werden kann. Unter der Textur der Gruppe von Bildpunkten ist bspw. eine Intensitätsverteilung (Helligkeitsstufen/Grauwerte) der Bildpunkte zu verstehen, aus der - je nach Größe der Gruppe - Umrisslinien oder dgl. erkennbar sein können. Sowohl die Intensität des interessierende Bildpunkts als auch die Intensitätsverteilung des Fensters wird beim Bestimmen (und Anwenden) der Zuordnung berücksichtigt.

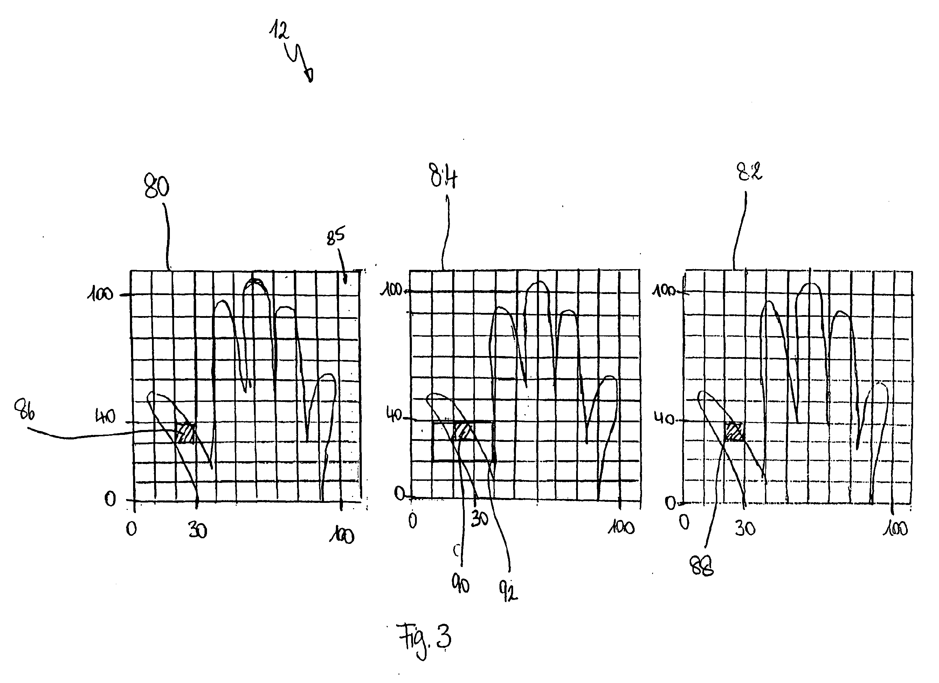

Ein dritter Eintrag 64 (der Eingabe 32) stellt u.a. registriere Koordinaten 68, eine weitere Gruppe von zusammengehörenden Bildpunkten 70 und/oder eine Klassenzugehörigkeit 72 dar, die jeweils einzeln oder auch miteinander kombiniert verwendet werden können. Der dritte Eintrag 64 repräsentiert also eine Vielzahl von weiteren Informationsquellen, auf die zurückgegriffen werden kann. Die hier aufgezählten Beispiele sind nicht abschließend. Es versteht sich, dass mehrere weitere Informationsquellen gleichzeitig berücksichtigt werden können. In diesem Fall kämen also vierte, fünfte, sechste, usw. Einträge dazu, die hier in Fig. 3 unter den dritten Einträgen 64 zusammengefasst erläutert werden.

Eine "registrierte Koordinate" erhält man dadurch, dass das zumindest erste Bild gegebene Bild und gegebenenfalls weitere Bilder mit einem oder mehreren Referenzbildern, die mit den gleichen Modalitäten aufgenommen wurden, registriert werden. Dazu wird eine lineare (Translation, Rotation, Skalierung) und/oder eine nicht-lineare Abbildung bestimmt, die jedem Punkt des zumindest ersten Bildes einen Punkt im Referenzbild zuordnet. Die "registrierte Koordinate" eines Punktes im gegebenen Bild ist die Koordinate des zugeordneten Bildpunktes im Referenz-Bild. Sie wird angegeben bezüglich eines vom Referenzbild abhängigen Koordinatensystems. Alternativ zu einem im allgemeinen dreidimensionalen Koordinatensystem, kann man die Position des zum interessierenden Punkt korrespondieren Punkt im Referenzbild auch relativ zu anatomisch charakteristischen Positionen im Referenzbild beschreiben, etwa der Nasenspitze.

In einer besonderen Ausgestaltung des vorliegenden Verfahrens können auch mehrere Referenzbilder verwendet werden, insbesondere können zum Beispiel verschiedene Referenzbilder für verschiedenen Geschlechter oder verschiedene Altersklassen verwendet werden. Auch für verschiedene anatomische Teilstücke können separate Referenzbilder verwendet werden.

Auch bei kleinen Fehlern bei der Registrierung - wie sie vorkommen können - kann die registrierte Koordinate als zusätzliche Information die Genauigkeit der Vorhersage signifikant erhöhen. Da zum Beispiel im MR-Bild Luft und Knochen die gleiche Intensität aufweisen und der Schwächungswert (für Knochen hoch, für Luft niedrig) daraus nicht eindeutig bestimmt werden kann, hilft oftmals schon eine ungenaue Ortsinformation: In der Lunge ist eher von Luft auszugehen, in der Nähe des Schädels von Knochen.

Auch außerhalb des gegebenen Bilds kann eine registrierte Koordinate bestimmt werden, da die Transformation, die das gegebene Bild auf das Referenz-Bild abbildet, sich stetig fortsetzen lässt (zum Beispiel sind linear Transformationen im gesamten Raum anwendbar).

Die Information der räumlichen Lage der Bildpunkte erhöht die Genauigkeit beim Zuordnen, so dass ein falsches Lernen oder Anwenden der Zuordnung zumindest minimiert oder vollständig ausgeschlossen werden kann. So ist es z.B. wichtig zu wissen, wo sich der beliebige Punkt ungefähr befindet. Betrachtet man z.B. den kleinen Finger der linken Hand (auf dem unbekannten Bild), so liegt dieser sicherlich innerhalb des größeren (räumlich) Bereichs der linken Hand. Diese (grobe) Information kann für die Lernmaschine und eine spätere Anwendung einer gelernten Zuordnung äußerst wichtig sein, insbesondere dann, wenn es (mit dem Finger) vergleichbare Referenzpaare gibt, die jedoch z.B. aus Bildern von Füßen extrahiert wurden. Die registrierten Koordinaten stellen also eine weitere, sehr hilfreiche Informationsquelle beim Bestimmen eines Eigenschaftswerts eines Bildpunkts aus einem (unbekannten) Bild dar.

Eine weitere Gruppe von Bildpunkten 70 kann aus weiteren Bildern bereitgestellt werden, die unter entweder den gleichen oder anderen Aufnahmemodalitäten wie das Bild erstellt wurden, aus dem die Gruppe 67 von Bildpunkten des zweiten Eintrags 62 extrahiert wurden. Ferner weist das weitere Bild (zumindest teilweise) einen gleichen Gegenstandsausschnitt wie die Gruppe der Bildpunkte des ersten Bildes auf.

Die weitere Gruppe von Bildpunkten stellt z.B. ein weiteres Fenster, einen Bildausschnitt oder ein ganzes Bild dar. Bspw. kann das zweite Bild - bei einem Verfahren zum Bestimmen der Schwächungskarte für ein PET-Bild basierend auf einem MR-Bild (erstes Bild) - ein weiteres MR-Bild oder ein PET-Bild, insbesondere das unkorrigierte PET-Bild sein. Das weitere MR-Bild kann bspw. eine andere Gewichtung wie das erste Bild aufweisen (T₁- und T₂-Gewichtung von MR-Bildern), so dass eine weitere Information für das maschinelle Lernen vorliegt.

Das zweite Bild kann zusätzlich zur Registrierung des ersten Bildes mit dem zweiten verwendet werden. Dies ist insbesondere von Vorteil, wenn das erste Bild, z.B. ein MR-Bild, geometrische Verzerrungen aufweist, wie sie bei MR-Bildern technisch bedingt häufig auftreten. Falls das zweite Bild geometrisch exakt ist, können die geometrischen Verzerrungen des ersten Bildes somit einfach korrigiert werden. Das zweite Bild kann zusätzlich Erweiterung des ersten verwendet werden. Dies ist insbesondere dann von Vorteil, falls das erste Bild einen kleineren Bildausschnitt als das zweite Bild zeigt. In diesem Fall kann außerhalb des Bildausschnittes des ersten Bildes nur auf Grundlage der Informationen des zweiten Bildes Eigenschaftswerte, insbesondere Schwächungswerte, für den fehlenden Bereich des ersten Bildes vorhergesagt werdenEine Klassenzugehörigkeit 72 kann ebenfalls ein dritter Eintrag 64 sein. Sie kann je nach Gegenstand andere Eigenschaften des Gegenstands umfassen. Ist der Gegenstand bspw. ein Patient, so umfasst sie z.B. ein Patientenalter, ein Patientengeschlecht, ein Patientengewicht und/oder eine Patientengewohnheit, wie z.B. Rauchen.

Weitere Einträge 66 der Eingabe 32 umfassen ein Intensitätshistogramm 74 und/oder eine Eigenschaft des Bildpunkts bezüglich der Gruppe von Bildpunkten 67, 76, wie z.B. ein Intensitätswert des interessierenden Bildpunkts relativ zu einem Intensitätsmittelwert von einem Teilbereich des Bilds oder bezüglich des Gesamtbildes.

Das Intensitätshistogramm 74 kann aus einer Intensitätsverteilung einer Untergruppe der Gruppe von zusammengehörenden Bildpunkten 67, 76 gebildet werden, die den interessierenden Bildpunkt aufweist. Bspw. kann die Untergruppe als Linie oder auch als Ring bzw. Kugelschale ausgewählt werden. Das Intensitätshistogramm stellt z.B. die Intensitätsverteilung in Abhängigkeit vom Abstand zum interessierenden Bildpunkts der Untergruppe dar.

Für den weiteren Eintrag 66 der Eingabe 32 wird basierend auf den Intensitätswerten der Gruppe von Bildpunkten, d.h. des Fensters, des Bildausschnitts oder des gesamten Bildes, ein Intensitätsmittelwert gebildet, der zusammen mit der Intensität des interessierenden Bildpunkts der Eingabe 32 hinzugefügt wird.

Die zweiten, dritten und weiteren Einträge können mitunter sehr viele verschiedene Informationen enthalten, die sich aber untereinander zum Teil sehr ähnlich sind. Anstatt alle Einträge zu speichern, kann man daher auch Gruppen von Einträgen, die sich untereinander besonders ähnlich sind, anhand der Trainingspaare herausfinden. Es reicht dann nur einen Index der Gruppe zu speichern anstatt alle einzelnen Einträge. Diese Indizes sind dann genauso informativ für die Zuordnung, die allerdings viel schneller und exakter durchgeführt werden kann. Diese Methoden sind bekannt unter dem Namen Dimensionalitätsreduktionsmethoden, insbesondere Hauptkomponentenanalyse oder einem Codebuch (Codebook). In einer bevorzugten Implementierung des beschrieben Verfahrens werden die berechneten Einträge für die Trainings- und Referenzpaare sowie für solche Paare, für die Eigenschaftswerte bestimmt werden sollen, mit Hilfe dieser Methoden komprimiert. Formal gesehen, werden komprimierten Einträge berechnet und diese der Struktur als weiteren Eintrag hinzugefügt. Der Lemalgorithmus kann dann gegebenenfalls nur diesen neuen Eintrag für eine Zuordnung in Betracht ziehen.

Die Vorhersage des unbekannten Eigenschaftswerts des beliebigen Punkts des ersten Bildes erfolgt also basierend auf einem oder mehreren bekannten Einträgen des beliebigen Punkts. Wird das Vorhersagen, lediglich bildhaft gesprochen, durch ein Vergleichen der bekannten Einträge (z.B. Intensität/Muster in einem Fenster) des beliebigen Punkts mit den Einträgen der Referenzpaare durchgeführt, so kann, falls einem Eintrag des beliebigen Punkts mehrere Eingaben von verschiedenen Referenzpaaren ähnlich gut entsprechen, die Anzahl dieser ähnlich guten Referenzpaare basierend auf den anderen bekannten Einträgen des beliebigen Punkts reduziert werden, um den richtigen Eigenschaftswert zu bestimmen.

Die Vorhersage des unbekannten Eigenschaftswerts des beliebigen Punkts kann im Wesentlichen basierend auf den zweiten Einträgen 62 erfolgen. Hierbei wird die Intensität des Punkts/Bildpunkts sowie die Textur der Gruppe von Bildpunkten berücksichtigt. Stellen die dritten Einträge 64 die registrierte Koordinate 68 dar, so können diese beim Vorhersagen des unbekannten Eigenschaftswerts berücksichtigt werden, um die Zuordnungsgenauigkeit zu erhöhen. Weist der beliebige Punkt (mit unbekannter Eigenschaft) keinen zweiten Eintrag 62 auf, so erfolgt die Vorhersage des unbekannten Eigenschaftswerts des beliebigen Punkts basierend auf einem oder mehreren der bekannten Einträge 64, 66 des beliebigen Punkts.

In Fig. 3 wird beispielhaft das Verfahren 12 für ein unkorrigiertes PET-Bild 80 dargestellt, dessen Schwächungskarte 82 basierend auf einem MR-Bild 84 bestimmt werden soll. Das PET-Bild 80 und das MR-Bild 84 werden bspw. durch einen PET-MR-Scanner bereitgestellt und zeigen Aufnahmen einer linken Hand eines rauchenden, 40-jährigen Patienten. Die (in diesem Beispiel nur) zweidimensionalen Bilder 80, 84 sind, schematisch durch allgemeine rechtwinklige Punkte 85 angedeutet, durch Pixel definiert, die hier sehr grob und in der Praxis bedeutend feiner sind. Mittels des Verfahrens der Erfindung soll für ein bestimmtes Pixel 86 des PET-Bildes 80 ein entsprechender Schwächungswert (Zahlenwert) 88 bestimmt werden. Hierzu wird ein bezüglich seiner räumlichen Lage dem Pixel 86 des PET-Bilds 80 entsprechendes Pixel 90 aus dem MR-Bild 84 ausgewählt und Fenster 92 um dieses Pixel 90 bestimmt. Das Fenster 92 weist außer dem Pixel 90 noch fünf weitere, direkt benachbarte Pixel auf. Das Fenster 92 stellt den zweiten Eintrag 62 (vgl. Fig. 2) der Eingabe 32 (vgl. Fig. 1) für die Lernmaschine dar. Als dritter Eintrag 64 der Eingabe 32 kann bspw. die registrierte Koordinate 68 des Pixels 90 verwendet werden, die in diesem Beispiel dem Wertepaar (30/40) entspricht. Hierzu wurde das MR-Bild 84 mit einem Referenzbild registriert, d.h. das MR-Bild 84 wurde mittels einer Transformation in dem Referenz-bild ausgerichtet. Als weitere Einträge 64 können die Klassenzugehörigkeiten des Patienten, d.h. sein Geschlecht (männlich), sein Alter (40 Jahre) und seine Lebensgewohnheit (Rauchen), verwendet werden. Bei der Zuordnung werden also keine Trainingspaare/Refererenzpaare berücksichtigt werden, die anhand von z.B. einer 60-jährigen Nichtraucherin gewonnen wurden. So kann z.B. berücksichtigt werden, dass Knochen mit dem Alter spröder werden, d.h. eine geringe Dichte aufweisen und somit in einer geringen Schwächung resultieren. Mittels einer zuvor anhand von Trainingspaaren mit identischer Struktur gelernten Zuordnung kann nun für den Pixel 90, bzw. für den Pixel 86, der zugehörige Schwächungswert 88 basierend auf den Einträgen des Pixels 90 vorhergesagt werden. Im obigen Erläuterungsbild gesprochen, beruht die Vorhersage auf einem Zuordnen (z.B. Vergleichen) der Eingabe 32 des Pixels 90 mit den Eingaben der Referenzpaare. An Hand der Einträge 62, 64 des Pixels 90 alle Referenzpaare ausgewählt werden, deren Einträge zu zumindest einem Eintrag 62, 64 des Pixels 90 ähnlich sind. Es wird dann aus diesen Referenzpaaren das bestübereinstimmende Referenzpaar ausgewählt, bei dem alle Einträge den Einträgen des Pixels 90 entsprechen. Der erste Eintrag dieses Referenzpaars wird dann dem Schwächungswert des Pixels 88 zugeordnet. In der Praxis wird der Schwächungswert mit der Lernmaschine berechnet, was sehr viel komplexer als der hier bildhaft beschriebene Vergleich ist und in der Regel auch nicht mit einem Vergleich identisch ist.

Wird die Schwächungskarte 82 aus CT-Bildern bereitgestellt, so muss diese, wie bspw. in P. E. Kinahan et al., "Attenuation correction for a combined 3D Pet/CT scanner", Med. Phys. 25(19, 1998 offenbart, auf die entsprechende Schwächungskarte für PET-Bilder umgerechnet werden.

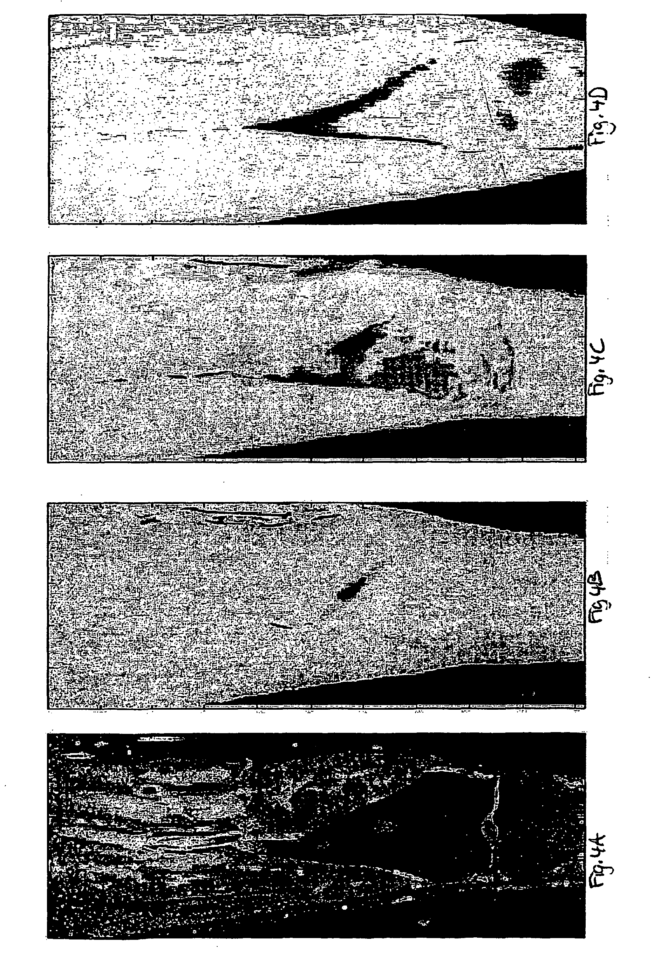

Fig. 4A - 4D veranschaulichen ein Beispiel für die unterschiedliche Genauigkeit des Verfahrens gemäß der vorliegenden Erfindung basierend auf den verwendeten Informationen. Fig. 4A zeigt einen Ausschnitt des MR-Bildes wie in Fig. 1 und stellt somit ein aufgenommenes MR-Bild dar, für das eine Schwächungskarte gelernt werden soll. Wird diese Schwächungskarte unter Verwendung von Fenstern mittels z.B. von Supported-Vektor-Regression bereitgestellt (Fig. 4B), so besitzt sie im Vergleich zu einer Schwächungskarte, für die sowohl Fenster als auch die registrierten Koordinaten verwendet werden (Fig. 4C), eine geringere Genauigkeit. Fig. 4D zeigt zum Vergleich die basierend auf CT-Bildern bestimmte Schwächungskarte.

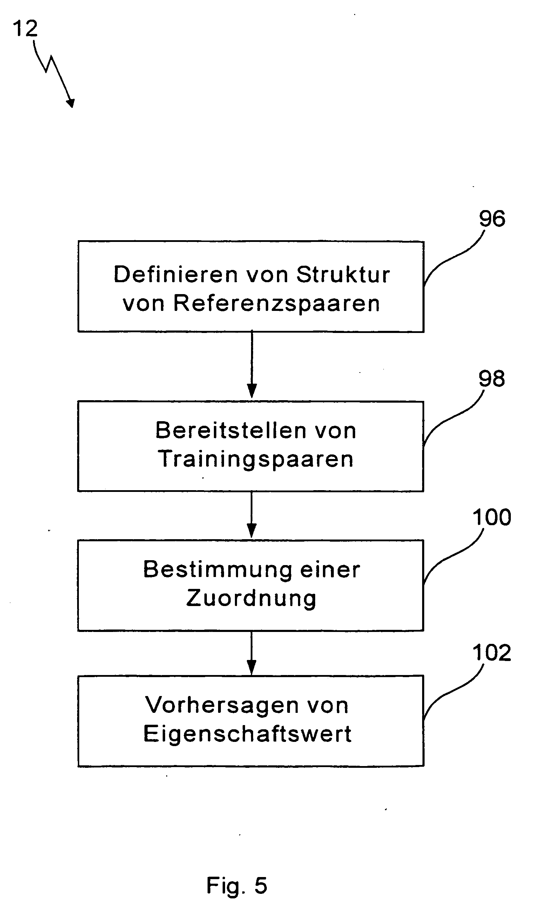

Fig. 5 zeigt ein Flussdiagram 94 des erfindungsgemäßen Verfahrens 12. In einem ersten Verfahrensschritt 96 wird die Struktur der Referenzpaare definiert, die die Eingabe(n) und Ausgabe für die Lernmaschine in Form von Einträgen aufweist. In einem nächsten Verfahrensschritt 98 wird eine Vielzahl von Trainingspaaren bereitgestellt, deren Struktur der Struktur der Referenzpaare entspricht. Es wird in einem weiteren Verfahrensschritt 100 mittels maschinellen Lernens die Zuordnung zwischen der Eingabe und der Ausgabe der Trainingspaare bestimmt, welche dann in einem weiteren Verfahrensschritt 102 auf den beliebigen Punkt angewendet wird, um den unbekannten Eigenschaftswert des beliebigen Punkts basierend auf den bekannten Einträgen seiner Eingabe vorauszusagen.

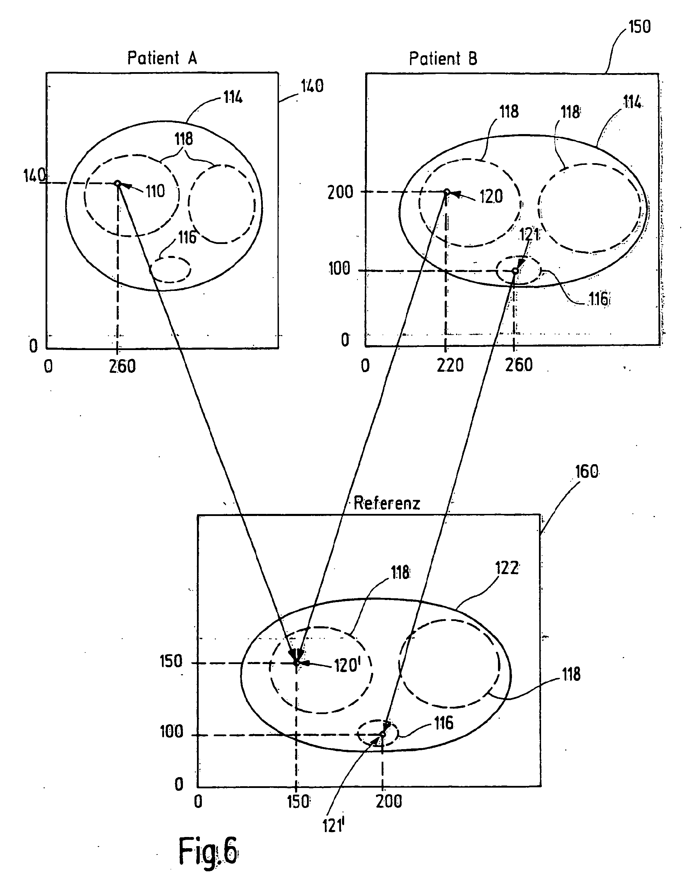

Bezugnehmend auf Fig. 6 ist nochmals ein Arbeiten mit registrierten Koordinaten beispielhaft gezeigt.

Gelegentlich beinhalten weder die Intensität eines einzelnen interessierenden Punktes noch ein Fenster um den interessierenden Punkt genügend Informationen, um einen Eigenschaftswert des interessierenden Punktes eindeutig zu bestimmen. Zum Beispiel könnte ein MR-Bildpunkt vollkommen dunkel sein, weil er einen Punkt in Luft zeigt, oder weil der Punkt komplett in einem Knochen liegt. Gleiches gilt für ein Fenster um diesen Punkt. Es kann also erforderlich sein, dass die Lernmaschine weitere Eingabe-Informationen braucht. Hier können registrierte Koordinaten weiterhelfen.

In der oberen Hälfte der Fig. 6 sind schematisch zwei Bilder 140 und 150 gezeigt, die jeweils einen Schnitt durch einen Patientenkörper (jeweils 114) im Brustbereich zeigen, wobei die Patienten verschieden sind und jeweils die Lunge 118 und die Wirbelsäule 116 zu erkennen ist. Ein (interessierender) Punkt 110 im ersten Bild 140 liegt mittig in der Lunge 118. Im Bild 140 weist er z.B. die Koordinaten140/260 auf. Der anatomisch korrespondierende Punkt ist der Punkt 120 im Bild 150 mit den Koordinaten 200/220. In der unteren Hälfte der Fig. 6 ist ein Bild 160 eines "Referenzmenschen" 122 gezeigt.

Nehmen wir an, dass für das zweite Bild 150 die Eigenschaftskarte bekannt ist und aus diesem Bild Trainings-/Referenzpaare gewonnen werden. Insbesondere seien zwei Referenzpaare an den Punkten 120 und 121 entnommen. Bei beiden ist das Fenster dunkel, da Luft in der Lunge und Knochen in der Wirbelsäule im MR-Bild jeweils kein Signal erzeugen; der zugehörige Eigenschaftswert, hier zum Beispiel ein PET-Schwächungswert, ist jedoch stark verschieden, hoch für Knochen und niedrig für Luft.

Soll nun für den Punkt 110 in Bild 140 der unbekannte Schwächungswert bestimmt werden, so werden die zweiten und weitere Einträge der Datenstruktur mit den entsprechenden Einträgen der Referenzpaare verglichen, um die Vorhersage - vereinfachende gesprochen - anhand des ähnlichsten Paares zu erbringen. Da sowohl das Fenster um den Punkt 110 im Bild 140 sowie die Fenster der Referenzpaare im Bild 150 dunkel sind, kann man anhand dieser Information nicht entscheiden, welches Referenzpaar ähnlicher ist. Vergleicht man die Koordinaten der des interessiernenden Punktes 110 (140/160) im ersten Bild 140 direkt mit denen des Referenzpaares am Ort 120 (200/220) im zweiten Bild 150, so sind diese zuerst einmal nicht sehr ähnlich, obwohl sie anatomisch den gleichen Punkt bezeichnen. Die Wahl eines der zwei Referenzpaare anhand dieser Koordinaten wäre zufällig und würde im gezeigten Beispiel fehlschlagen, d.h. der Punkt 121 mit den Koordinaten (100/260) würde gewählt werden. Dadurch wäre der vorhergesagte Schwächungswert falsch (hoch).

Durch Verwendung der registrierten Koordinate (150/150) für den interessierenden Punkt, sowie entsprechend für die Referenzpaare (150/150) und (100/200) und wird die Zuordnung jedoch eindeutig. Das Referenzpaar am Ort 120 hat die gleichen registrierten Koordinaten (150/150) wie der interessierende Punkt 110 und ist somit ähnlicher als das zweite Referenzpaar am Ort 121 mit den registrierten Koordinaten (100/200). Der vorhergesagte Schwächungswert (niedrig) ist jetzt korrekt.

Es versteht sich, dass die obigen Ausführungen wieder nur einer bildhaften Veranschaulichung dienen und nicht einschränkend auszulegen sind.

Ferner sei erwähnt, dass für jeden interessierenden Punkt der Eigenschaftswert mittels einer Atlas-Registrierung bestimmt wird, und dieser Wert der Struktur als weiterer Eintrag hinzugefügt werden kann.

Dieser Aspekt macht das erfindungsgemäße Verfahren den bekannten Atlasverfahren überlegen. Bei den bekannten Atlasverfahren ist einzig und allein die registrierte Koordinate ausschlaggebend für den resultierenden Schwächungswert. Die tatsächliche Intensität interessiert nicht mehr, da diese nur in einem ersten Schritt benötigt wird, um sich innerhalb des Atlas "einzupeilen", wobei nicht nur ein Bildpunkt (mit seiner Intensität), sondern alle Bildpunkte des Bilds benötigt werden. Weist das Bild zu starke Anomalien auf, so ist im Stand der Technik schon des "Einpeilen" im Atlas erfolglos. Gemäß der vorliegenden Erfindung stellen Anomalien jedoch kein Problem dar.

## Patentansprüche

1. Verfahren zum Bestimmen einer Eigenschaftskarte (82) für einen Gegenstand, insbesondere für ein Lebewesen, basierend auf zumindest einem ersten Bild (84), nämlich einem Kernspinresonanz-Bild, MR-Bild, des Gegenstands, mit folgenden Schritten:
- Definieren einer Struktur für Referenzpaare, wobei jedes Referenzpaar zumindest zwei Einträge (62) aufweist, wobei der erste Eintrag einen Eigenschaftswert, nämlich einen Schwächungswert, darstellt, und
wobei der zweiten Eintrag (62) eine Gruppe von zusammengehörenden Bildpunkten (67), die dem MR-Bild (28) extrahiert sind, darstellt, wobei die Gruppe zumindest einen dem Eigenschaftswert entsprechenden interessierenden Bildpunkt umfasst;
- Bereitstellen einer Vielzahl von Trainingspaaren (16 - 26), wobei eine Struktur der Trainingspaare (16 - 26) der Struktur der Referenzpaare (16 - 26) entspricht, und wobei die Einträge (62 - 66) der jeweiligen Trainingspaare (16 - 26) bekannt sind (98);
- Bestimmen einer Zuordnung zwischen den ersten Einträgen und den weiteren Einträgen (62 - 66) der Trainingspaare (16 - 26) durch maschinelles Lernen (100), um so für einen beliebigen Bildpunkt (90) des ersten Bildes (84) einen entsprechenden Eigenschaftswert (88) vorhersagen zu können (102),
- wobei die Struktur der Referenzpaare zusätzlich einen dritten Eintrag (64) aufweist, der beim Bestimmen der Zuordnung zum ersten Eintrag zusammen mit den zweiten Eintrag (62) berücksichtigt wird,
- wobei der dritte Eintrag (64) eine weitere Gruppe von zusammengehörenden Bildpunkten (70) eines zumindest zweiten Bildes (80), nämlich eines PET-Bildes, darstellt, wobei die weitere Gruppe von Bildpunkten (70) des zumindest zweiten Bildes (80) zumindest teilweise einen gleichen Gegenstandsausschnitt wie die Gruppe der Bildpunkte des ersten Bildes (84) zeigt, und
- wobei zusätzlich das zumindest zweite Bild (80) für eine Registrierung mit dem zumindest ersten Bild (84) und zum Erweitern des zumindest ersten Bildes (84) verwendet wird um so, falls das erste Bild einen kleineren Gegenstandsausschnitt als das zweite Bild zeigt, die Eigenschaftskarte für das gesamte zweite Bild bereit zu stellen.

2. Verfahren nach Anspruch 1, wobei weitere Einträge (64) darstellen:
- eine registrierte Koordinate (68); und/oder
- eine Klassenzugehörigkeit (72).

3. Verfahren nach Anspruch 1, wobei der zweite Eintrag eine registrierte Koordinate darstellt.

4. Verfahren nach Anspruch 2 oder 3, wobei die registrierte Koordinate durch eine Koordinate eines zu dem interessierenden Punkt in einem Referenzgegenstand äquivalenten Punkt, und/oder durch einen oder mehrere Abstände des zu dem interessierenden Punkt äquivalenten Punkt relativ zu anatomisch charakteristischen Positionen im Referenzgegenstand definiert ist.

5. Verfahren nach einem der Ansprüche 2 bis 4, wobei eine Ähnlichkeit zwischen der registrierten Koordinate mit einem ortsabhängigen Abstandsmaß bestimmt werden, das von Eigenschaften des Referenzgegenstands sowie einer Position des äquivalenten Punktes im Referenzgegenstand abhängt.

6. Verfahren nach Anspruch 2, wobei die Klassenzugehörigkeit (72) umfasst: ein Patientenalter, ein Patientengeschlecht, ein Patientengewicht und/oder eine Patientenlebensgewohnheit.

7. Verfahren nach einem der Ansprüche 1 bis 6, wobei aus der Gruppe von zusammengehörenden Bildpunkten (36 - 46, 92) eine Untergruppe ausgewählt wird, die den interessierenden Bildpunkt (90) aufweist.

8. Verfahren nach einem der Ansprüche 1 bis 7, wobei jeder Bildpunkt (90) einen Intensitätswert aufweist.

9. Verfahren nach einem der Ansprüche 1 bis 8, wobei zu jeder Gruppe von zusammengehörenden Bildpunkten (36 - 46, 92) eine Textur bestimmt wird.

10. Verfahren nach Anspruch 7 und 8, wobei basierend auf der Untergruppe ein Intensitätshistogramm (74) erstellt wird, das der Struktur als weiterer Eintrag (66) hinzugefügt werden kann.

11. Verfahren nach einem der Ansprüche 1 bis 10, wobei basierend auf den Intensitätswerten zumindest der Bildpunkte der Gruppe von zusammengehörenden Bildpunkten (36 - 46) ein Intensitätsmittelwert (76) gebildet wird, der zusammen mit der Intensität des interessierenden Bildpunkts als weiterer Eintrag (66) der Struktur hinzugefügt wird.

12. Verfahren nach einem der Ansprüche 1 bis 8, wobei für den beliebigen Punkt (90) des Gegenstand eine Struktur, die der Struktur der Referenzpaare entspricht, bereitgestellt wird, und wobei die Struktur des beliebigen Punkts (90) zumindest einen von dem ersten Eintrag verschiedenen bekannten Eintrag (62
- 66) aufweist und der erste Eintrag fehlt.

13. Verfahren nach Anspruch 12, wobei das Vorhersagen des ersten Eintrags des beliebigen Punkts (90) durch Anwenden der gelernten Zuordnung basierend auf zumindest einem oder mehreren der bekannten Einträge (62 - 66) des beliebigen Punkts (90) erfolgt.

14. Verfahren nach Anspruch 13, wobei die Vorhersage, wenn für den beliebigen Punkt (90) kein zweiter Eintrag (62) bekannt ist, basierend auf einem oder mehreren bekannten weiteren Einträgen (63 - 66, 68 - 76) erfolgt.

15. System zum Bestimmen einer Eigenschaftskarte für einen Gegenstand, insbesondere für ein Lebewesen, basierend auf zumindest einem ersten Bild, nämlich, einem Kernspinresonanzbild, MR-Bild, des Gegenstands, mit:
- Mitteln zum Definieren einer Struktur für Referenzpaare, wobei jedes Referenzpaar zumindest zwei Einträge aufweist, wobei der erste Eintrag einen Eigenschaftswert, nämlich einen Schwächungswert, darstellt, und wobei der zweite Eintrag eine Gruppe von zusammengehörigen Bildpunkten die aus dem MR-Bild extratiert sind, darstellt, die einen dem Eigenschaftswert entsprechenden interessierenden Bildpunkt umfasst;
- Mittel zum Einlesen einer Vielzahl von Trainingspaaren; und
- einer Lernmaschine zum Bestimmen einer Zuordnung zwischen den ersten Einträgen und den weiteren Einträgen der Trainingspaare, um für einen beliebigen Bildpunkt des ersten Bildes einen entsprechenden Eigenschaftswert vorhersagen zu können,
- wobei die Struktur der Referenzpaare zusätzlich einen dritten Eintrag (64) aufweist, der beim Bestimmen der Zuordnung zum ersten Eintrag zusammen mit den zweiten Eintrag (62) berücksichtigt wird,
- wobei der dritte Eintrag (64) eine weitere Gruppe von zusammengehörenden Bildpunkten (70) eines zumindest zweiten Bildes (80), nämlich eines PET-Bildes, darstellt, wobei die weitere Gruppe von Bildpunkten (70) des zumindest zweiten Bildes (80) zumindest teilweise einen gleichen Gegenstandsausschnitt wie die Gruppe der Bildpunkte des ersten Bildes (84) zeigt, und
- wobei zusätzlich das zumindest zweite Bild (80) für eine Registrierung mit dem zumindest ersten Bild (84) und zum Erweitern des zumindest ersten Bildes (84) verwendet wird um so, falls das erste Bild einen kleineren Gegenstandsausschnitt als das zweite Bild zeigt, die Eigenschaftskarte für das gesamte zweite Bild bereit zu stellen.

## Claims

1. A method for determining a property map (82) of an object, particularly a living being, based on at least a first image (84), namely a magnetic resonance image, MR image, of the object, comprising the following steps:
- defining a structure of reference pairs, wherein each reference pair comprises at least two entries (62), wherein the first entry represents a property value, namely an attenuation value, and wherein the second entry (62) represents a group of image points belonging together (67), which are extracted from the MR image (28), wherein the group includes at least one interesting image point which corresponds to the property value;
- providing a plurality of training pairs (16 - 26), wherein a structure of the training pairs (16 - 26) corresponds to the structure of reference pairs (16 -26), and wherein the entries (62 - 66) of the respective training pairs (16 - 26) are known (98);
- determining an assignment between the first entries and the other entries (62 - 66) of the training pairs (16 - 26) by machine learning (100) allowing to predict (102) in this manner a corresponding property value (88) for an arbitrary image point (90) of the first image (84),
- wherein the structure of reference pairs additionally comprises a third entry (64), which is taken into account during determination of the assignment to the first entry, together with the second entry (62),
- wherein the third entry (64) represents another group of image points belonging together (70) of at least a second image (80), namely a PET image, wherein the other group of image points (70) of the at least second image (80) shows at least partially an identical part of the object like the group of image points of the first image (84), and
- wherein additionally the at least second image (80) is used for a registration with the at least first image (84) and is used for extending the at least first image (84), in order to provide in this manner, if the first image shows a smaller part of the object than the second image, the property map of the entire second image.

2. A method according to claim 1, wherein further entries (64) represent:
- a registered coordinate (68); and/or
- a class affiliation (72).

3. A method according to claim 1, wherein the second entry represents a registered coordinate.

4. A method according to claim 2 or 3, wherein the registered coordinate is defined by the coordinate of a point that is equivalent to the interesting point in a reference object, and/or one or more distances of the point that is equivalent to the interesting point relative to anatomically characteristic positions in the reference object.

5. A method according to claim 2 to 4, wherein a similarity between the registered coordinate is determined with a position-dependent distance measure, which depends on properties of the reference object and a position of the equivalent point in the reference object.

6. A method according to claim 2, wherein the class affiliation (72) comprises: a patient's age, a patient's sex, a patient's weight, and/or a patient's habit.

7. A method according to claim 1 to 6, wherein a subgroup is selected from the group of image points belonging together (36 - 46, 92), which comprises the interesting image point (90).

8. A method according to claim 1 to 7, wherein each image point (90) has an intensity value.

9. A method according to claim 1 to 8, wherein a texture is determined for each group of image points belonging together (36 - 46, 92).

10. A method according to claims 7 and 8, wherein based on the subgroup an intensity histogram (74) is established, which can be added to the structure as another entry (66).

11. A method according to claims 1 to 10, wherein based on the intensity values of at least the image points of the group of image points belonging together (36 - 46) an intensity average is formed, which is added to the structure together with the intensity of the interesting image points as another entry (66).

12. A method according to claims 1 to 8, wherein a structure is provided for the arbitrary point (90) of the object, which corresponds to the structure of the reference pairs, and wherein the structure of the arbitrary point (90) comprises at least one entry (62 - 66) which is known and different to the first entry, and wherein the first entry is missing.

13. A method according to claim 12, wherein the prediction of the first entry of the arbitrary point (90) is performed by applying the learnt assignment based on at least one or more of the known entries (62 - 66) of the arbitrary point (90).

14. A method according to claim 13, wherein the prediction, if no second entry (62) is known for the arbitrary point (90), is performed based on one or more known additional entries (63 - 66, 68 - 76).

15. A system for determining a property map of an object, particularly a living being, based on at least a first image, namely a magnetic resonance image, MR image, of the object, comprising:
- means for defining a structure of reference pairs, wherein each reference pair has at least two entries, wherein the first entry represents a property value, namely an attenuation value, and wherein the second entry represents a group of image points belonging together, which are extracted from the MR image, which comprises an interesting point corresponding to the property value;
- means for reading a plurality of training pairs; and
- a learning machine for determining an assignment between the first entries and the other entries of the training pairs, allowing prediction of a property value corresponding to an arbitrary image point of the first image,
- wherein the structure of reference pairs additionally comprises a third entry (64), which is taken into account during determination of the assignment to the first entry, together with the second entry (62),
- wherein the third entry (64) represents another group of image points belonging together (70) of at least a second image (80), namely a PET image, wherein the other group of image points of the at least second image (80) shows at least partially an identical part of the object like the group of image points of the first image (84), and
- wherein additionally the at least second image (80) is used for a registration with the at least first image (84) and is used for extending the at least first image (84), in order to provide in this manner, if the first image shows a smaller part of the object than the second image, the property map of the entire second image.

## Revendications

1. Procédé de détermination d'une carte de propriétés (82) pour un objet, en particulier pour un être vivant, sur la base d'au moins une première image (84), à savoir une image par résonance magnétique nucléaire, ou image RM, de l'objet, comprenant les étapes suivantes :
- définition d'une structure pour des paires de référence, chaque paire de référence comportant au moins deux entrées (62), la première entrée représentant une valeur de propriété, à savoir une valeur d'atténuation, et la deuxième entrée (62) représentant un groupe de points d'image liés entre eux (67) qui sont extraits de l'image RM (28), le groupe comprenant au moins un point d'image d'intérêt correspondant à la valeur de propriété ;
- préparation d'une pluralité de paires d'apprentissage (16 - 26), une structure des paires d'apprentissage (16 - 26) correspondant à la structure des paires de référence (16, 26), et les entrées (62 - 66) des paires d'apprentissage respectives (16 - 26) étant connues (98) ;
- détermination d'une relation entre les premières entrées et les entrées supplémentaires (62 - 66) des paires d'apprentissage (16 - 26) par apprentissage automatique (100), pour ainsi pouvoir prédire (102) une valeur de propriété correspondante (88) pour un point d'image arbitraire (90) de la première image (84) ;
- dans lequel la structure des paires de référence comporte en outre une troisième entrée (64) qui est prise en compte lors de la détermination de la relation à la première entrée en association avec la deuxième entrée (62) ;
- dans lequel la troisième entrée (64) représente un groupe supplémentaire de points d'image liés entre eux (70) d'au moins une deuxième image (80), à savoir une image PET, le groupe supplémentaire de points d'image (70) de ladite au moins une deuxième image (80) présentant au moins en partie une même section transversale de l'objet que le groupe de points d'image de la première image (84), et
- dans lequel ladite au moins une deuxième image (80) est utilisée pour un alignement avec ladite au moins une première image (84) et pour l'agrandissement de ladite au moins une première image (84) afin de préparer la carte de propriétés pour la totalité de la deuxième image dans le cas où la première image présente une section transversale de l'objet plus petite que la deuxième image.

2. Procédé selon la revendication 1, dans lequel des entrées supplémentaires (64) représentent :
- une coordonnée alignée (68) ; et/ou
- un critère de classification (72).

3. Procédé selon la revendication 1, dans lequel la deuxième entrée représente une coordonnée alignée.

4. Procédé selon la revendication 2 ou 3, dans lequel la coordonnée alignée est définie comme étant une coordonnée d'un point équivalent au point d'intérêt dans un objet de référence, et/ou comme étant une ou plusieurs distance(s) du point équivalent au point d'intérêt par rapport à des positions anatomiques caractéristiques dans l'objet de référence.

5. Procédé selon l'une des revendications 2 à 4, dans lequel une ressemblance entre les coordonnées alignées est déterminée au moyen d'une mesure de distance dépendant de la position qui dépend de propriétés de l'objet de référence ainsi que d'une position du point équivalent dans l'objet de référence.

6. Procédé selon la revendication 2, dans lequel le critère de classification (72) comprend : l'âge du patient, le sexe du patient, le poids du patient et/ou les habitudes de vie du patient.

7. Procédé selon l'une des revendications 1 à 6, dans lequel dans le groupe de points d'image liés entre eux (36 - 46, 92) un sous-groupe qui comporte le point d'image d'intérêt (90) est sélectionné.

8. Procédé selon l'une des revendications 1 à 7, dans lequel chaque point d'image (90) présente une valeur d'intensité.

9. Procédé selon l'une des revendications 1 à 8, dans lequel une texture est déterminée par rapport à chaque groupe de points d'image liés entre eux (36 - 46, 92).

10. Procédé selon les revendications 7 et 8, dans lequel, sur la base du sous-groupe, un histogramme d'intensité (74) est établi, lequel histogramme d'intensité peut être ajouté à la structure en tant qu'entrée supplémentaire (66).

11. Procédé selon l'une des revendications 1 à 10, dans lequel, sur la base des valeurs d'intensité d'au moins les points d'image du groupe de points d'image liés entre eux (36 - 46), une valeur moyenne d'intensité (76) est calculée, laquelle valeur moyenne d'intensité est ajoutée à la structure en association avec l'intensité du point d'image d'intérêt en tant qu'entrée supplémentaire (66).

12. Procédé selon l'une des revendications 1 à 8, dans lequel, pour le point arbitraire (90) de l'objet, une structure qui correspond à la structure de la paire de référence est préparée, et dans lequel la structure du point arbitraire (90) comporte au moins une entrée connue (62 - 66) différente de la première entrée et dans lequel la première entrée est absente.

13. Procédé selon la revendication 12, dans lequel la prédiction de la première entrée du point arbitraire (90) s'effectue par utilisation de la relation obtenue par apprentissage sur la base d'au moins une ou plusieurs des entrées connues (62 - 66) du point arbitraire (90).

14. Procédé selon la revendication 13, dans lequel la prédiction s'effectue sur la base d'une ou plusieurs entrées supplémentaires connues (63 - 66, 68 - 76) lorsqu'aucune deuxième entrée (62) n'est connue pour le point arbitraire (90).

15. Système de détermination d'une carte de propriétés pour un objet, en particulier pour un être vivant, sur la base d'au moins une première image, à savoir une image par résonance magnétique nucléaire, ou image RM, de l'objet, comprenant :
- des moyens de définition d'une structure pour des paires de référence, chaque paire de référence comportant au moins deux entrées, la première entrée représentant une valeur de propriété, à savoir une valeur d'atténuation, et la deuxième entrée représentant un groupe de points d'image liés entre eux qui sont extraits de l'image RM, lequel groupe comprend un point d'image d'intérêt correspondant à la valeur de propriété ;
- un moyen de lecture d'une pluralité de paires d'apprentissage ; et
- une machine d'apprentissage pour la détermination d'une relation entre les premières entrées et les entrées supplémentaires des paires d'apprentissage, pour pouvoir prédire une valeur de propriété correspondante, pour un point d'image arbitraire de la première image,
- dans lequel la structure des paires de référence comporte en outre une troisième entrée (64) qui est prise en compte lors de la détermination de la relation à la première entrée en association avec la deuxième entrée (62),
- dans lequel la troisième entrée (64) représente un groupe supplémentaire de points d'image liés entre eux (70) d'au moins une deuxième image (80), à savoir une image PET, le groupe supplémentaire de points d'image (70) de ladite au moins une deuxième image (80) présentant au moins en partie une même section transversale d'objet que le groupe de points d'image de la première image (84), et
- dans lequel en outre ladite au moins une deuxième image (80) est utilisée pour un alignement avec ladite au moins une première image (84) et pour l'agrandissement de ladite au moins une première image (84) afin de préparer la carte de propriétés pour la totalité de la deuxième image dans le cas où la première image présente une section transversale de l'objet plus petite que la deuxième image.
